# EUROPEAN PATENT APPLICATION

(11) **EP 4 699 525 A1**
(43) Date of publication of application: **25.02.2026**
(21) Application number: 24884802.0
(22) Date of filing: 30.10.2024
(51) Int. Cl.: A61B 5/021, A61B 5/022, A61B 5/00, A61B 5/024, A61B 5/0225

(54) **METHOD FOR GENERATING BLOOD PRESSURE MEASUREMENT SCHEME, ELECTRONIC DEVICE, AND STORAGE MEDIUM**

(30) Priority: 31.10.2023 CN 202311442868
(71) Applicant: Huawei Technologies Co., Ltd., Shenzhen, Guangdong 518129 (CN)
(72) Inventor: JIN, Junye, Shenzhen, Guangdong 518129 (CN); LI, Hongbao, Shenzhen, Guangdong 518129 (CN); FU, Bo, Shenzhen, Guangdong 518129 (CN); HUANG, Jiejing, Shenzhen, Guangdong 518129 (CN); LI, Dong, Shenzhen, Guangdong 518129 (CN); LU, Shiqiang, Shenzhen, Guangdong 518129 (CN)
(74) Representative: Grünecker Patent- und Rechtsanwälte PartG mbB
(86) International application number: PCT/CN2024/128432
(87) International publication number: WO 2025/092800

(57) **Abstract**

A method for generating a blood pressure measurement scheme, an electronic device, and a storage medium are disclosed. A wearable device (100) obtains one or more of user information, physiological data, and a device status. The wearable device generates a blood pressure measurement scheme based on one or more of the user information, the physiological data, and the device status, where the blood pressure measurement scheme includes one or more of the following: a start moment of blood pressure measurement, an end moment of blood pressure measurement, a duration of blood pressure measurement, a quantity of times of blood pressure measurement within the duration of blood pressure measurement, a time interval between two adjacent times of blood pressure measurement within the duration of blood pressure measurement, and a total quantity of times of blood pressure measurement. The wearable device obtains a blood pressure measurement value of a user within one blood pressure measurement cycle based on the blood pressure measurement scheme. The wearable device may automatically generate the blood pressure measurement scheme, so that the blood pressure measurement scheme generated by the wearable device better conforms to user characteristics. This not only simplifies a user operation, but also improves appropriateness of blood pressure measurement.

## Description

This application claims priority to Chinese Patent Application No. 202311442868.2, with the China National Intellectual Property Administration on October 31, 2023 and entitled "METHOD FOR GENERATING BLOOD PRESSURE MEASUREMENT SCHEME, ELECTRONIC DEVICE, AND STORAGE MEDIUM", which is incorporated herein by reference in its entirety.

### TECHNICAL FIELD

This application relates to the field of wearable technologies, and in particular, to a method for generating a blood pressure measurement scheme, an electronic device, and a storage medium.

### BACKGROUND

With improvement of living standards, people's health attracts increasing attention. Hypertension is a common cardiovascular disease, and regular blood pressure measurement is one of important means for ensuring health of patients with hypertension. Dynamic blood pressure measurement is a technology for continuously measuring blood pressure of a user for 24 hours without affecting daily activities of the user, and a plurality of blood pressure measurement values may be obtained within 24 hours. Usually, measurement is performed every 10 to 15 minutes, and an average value of the plurality of blood pressure measurement values within 24 hours is used as a blood pressure value. Currently, there is a cuff-based blood pressure monitor for measuring dynamic blood pressure of a user. However, to use the cuff-based blood pressure monitor, the user needs to carry the cuff-based blood pressure monitor for 24 hours. It is inconvenient for the user to use the cuff-based blood pressure monitor.

To facilitate blood pressure measurement for a user, a wearable device (for example, a watch) is equipped with a blood pressure detection component, and the user may wear the wearable device to implement dynamic blood pressure measurement. This saves time and effort. The user may set a blood pressure measurement scheme on the wearable device, for example, set blood pressure measurement time and a time interval between two adjacent times of blood pressure measurement. However, an operation of manually setting the blood pressure measurement scheme by the user is complex, and how to simplify the user operation is to be further studied.

### SUMMARY

This application provides a method for generating a blood pressure measurement scheme, an electronic device, and a storage medium, to enable a wearable device to automatically generate a blood pressure measurement scheme for a user, so that the blood pressure measurement scheme generated by the wearable device better conforms to characteristics and lifestyle habits of the user, to improve appropriateness of blood pressure measurement.

According to a first aspect, this application provides a method for generating a blood pressure measurement scheme. The method includes: A wearable device obtains one or more of user information, physiological data, and a device status. The wearable device generates a blood pressure measurement scheme based on one or more of the user information, the physiological data, and the device status, where the blood pressure measurement scheme includes one or more of the following: a start moment of blood pressure measurement, an end moment of blood pressure measurement, a duration of blood pressure measurement, a quantity of times of blood pressure measurement within the duration of blood pressure measurement, a time interval between two adjacent times of blood pressure measurement within the duration of blood pressure measurement, and a total quantity of times of blood pressure measurement. The wearable device obtains a blood pressure measurement value of a user within one blood pressure measurement cycle based on the blood pressure measurement scheme.

For example, one blood pressure measurement cycle may be 24 hours.

According to the method, the wearable device may automatically generate the blood pressure measurement scheme based on fusion of a plurality of types of information, so that the blood pressure measurement scheme generated by the wearable device better conforms to user characteristics. This not only simplifies a user operation, but also improves appropriateness of blood pressure measurement.

With reference to the first aspect, in a possible implementation, the user information includes one or more of the following: a gender, an age, a height, a weight, smoking history, alcohol consumption, medical history, medication history, and lifestyle habits; the physiological data includes one or more of the following: a heart rate, blood pressure, arteriosclerosis, and blood glucose; and the device status includes one or more of the following: a remaining battery level and device temperature.

With reference to the first aspect, in a possible implementation, that the wearable device generates the blood pressure measurement scheme based on one or more of the user information, the physiological data, and the device status specifically includes: The wearable device determines a risk level of a hypertension risk of the user based on one or more of the user information, the physiological data, and the device status. The wearable device generates the blood pressure measurement scheme based on the risk level of the hypertension risk of the user.

Different blood pressure measurement schemes are generated for different risk levels of hypertension risks. For example, a total quantity of times of blood pressure measurement that is determined by the wearable device based on a high risk is greater than a total quantity of times of blood pressure measurement that is determined by the wearable device based on a low risk.

In some embodiments, the risk level of the hypertension risk may be a risk level within a specific time period within one blood pressure measurement cycle. For example, a risk level of a hypertension risk of the user within a nighttime period is higher than a risk level of a hypertension risk of the user within a daytime period. In this case, a total quantity of times of blood pressure measurement within the nighttime period is greater than a total quantity of times of blood pressure measurement within the daytime period.

In this way, the wearable device may determine the risk level of the hypertension risk of the user based on a plurality of types of information, and then generate the blood pressure measurement scheme based on the risk level of the hypertension risk.

With reference to the first aspect, in a possible implementation, after the wearable device generates the blood pressure measurement scheme, the method further includes: The wearable device displays first prompt information, where the first prompt information is used to prompt the user to view detailed content of the blood pressure measurement scheme. The wearable device receives and responds to a first operation of the user for a first option in the first prompt information, and displays a first user interface, where the detailed content of the blood pressure measurement scheme is displayed on the first user interface.

In this way, after the wearable device automatically generates the blood pressure measurement scheme, the wearable device may prompt the user to view the detailed content of the blood pressure measurement scheme. In some embodiments, the wearable device may alternatively receive a user operation to modify the blood pressure measurement scheme.

With reference to the first aspect, in a possible implementation, before the wearable device obtains the blood pressure measurement value of the user within the blood pressure measurement cycle through measurement based on the blood pressure measurement scheme, the method further includes: The wearable device obtains the remaining battery level of the wearable device. The wearable device displays second prompt information when the remaining battery level of the wearable device is less than a preset battery level, where the second prompt information is used to prompt the user to charge the wearable device, and the preset battery level is a minimum battery level needed by the wearable device to complete blood pressure measurement within one blood pressure measurement cycle based on the blood pressure measurement scheme. The wearable device saves the blood pressure measurement scheme if the wearable device is in a charging state within a first duration after the second prompt information is displayed.

In this way, before the wearable device measures blood pressure based on the automatically generated blood pressure measurement scheme, the device needs to determine whether the remaining battery level of the wearable device is sufficient, to avoid a case in which the wearable device is powered off before blood pressure measurement within one cycle is completed. When the wearable device displays the prompt information and the wearable device is in the charging state, the wearable device may save the blood pressure measurement scheme, and complete blood pressure measurement within one blood pressure measurement cycle based on the blood pressure measurement scheme.

With reference to the first aspect, in a possible implementation, the method further includes: The wearable device displays third prompt information if the wearable device is not in the charging state within the first duration after the second prompt information is displayed, where the third prompt information is used to prompt the user to change the blood pressure measurement scheme. The wearable device receives and responds to a second operation of the user for a second option in the third prompt information, and obtains and saves a first updated blood pressure measurement scheme, where a total quantity of times of blood pressure measurement in the first updated blood pressure measurement scheme is less than the total quantity of times of blood pressure measurement in the blood pressure measurement scheme. The wearable device obtains a blood pressure measurement value of the user within one blood pressure measurement cycle based on the first updated blood pressure measurement scheme.

Optionally, a battery level needed for completing blood pressure measurement within one blood pressure measurement cycle based on the first updated blood pressure measurement scheme is less than or equal to the remaining battery level of the wearable device.

In this way, if the wearable device is still not in the charging state after the wearable device displays the prompt information, the wearable device may re-prompt the user to modify the blood pressure measurement scheme, and obtain the first updated blood pressure measurement scheme.

With reference to the first aspect, in a possible implementation, the method further includes: The wearable device receives and responds to a third operation of the user for a third option in the third prompt information, and displays fourth prompt information, where the fourth prompt information is used to prompt the user to set the wearable device to a low power mode. The wearable device receives and responds to a fourth operation of the user for a fourth option in the fourth prompt information, and enters the low power mode.

Optionally, in the low power mode, some or all of energy-consuming operations on the wearable device 100 that are irrelevant to blood pressure monitoring may be disabled. For example, the energy-consuming operations may include but are not limited to a heart rate monitoring function, a motion data recording function, and the like.

In this way, if the user still does not change the blood pressure measurement scheme after the wearable device displays the prompt information, the wearable device may re-prompt the user to control the wearable device to enter the low power mode, to save battery power of the device.

With reference to the first aspect, in a possible implementation, the method further includes: The wearable device receives and responds to a fifth operation of the user for a fifth option in the fourth prompt information, and displays fifth prompt information, where the fifth prompt information is used to notify the user that a battery level of the wearable device is excessively low.

In this way, if the wearable device still does not enter the low power mode after the wearable device displays the prompt information, the wearable device may notify the user that the battery level of the wearable device is excessively low and the wearable device may be powered off at any time.

With reference to the first aspect, in a possible implementation, the blood pressure measurement value includes a first blood pressure measurement value obtained by the wearable device at a first moment. After the wearable device obtains the first blood pressure measurement value, the method further includes: The wearable device obtains a current user status. When the current user status meets a blood pressure measurement condition, the wearable device saves the first blood pressure measurement value.

In a possible implementation, the method further includes: When the current user status does not meet the blood pressure measurement condition, the wearable device obtains a blood pressure compensation value based on the current user status. The wearable device obtains a first blood pressure monitoring value based on the blood pressure compensation value and the first blood pressure measurement value. The wearable device saves the first blood pressure monitoring value.

The blood pressure measurement condition may include but is not limited to one or more of the following: The user is in a stationary state, and a difference between a real-time heart rate and a nighttime resting heart rate is within a preset range.

The wearable device may collect motion data based on a motion sensor. The motion sensor may include but is not limited to an acceleration sensor, a gyroscope sensor, and the like. The motion sensor may collect motion data or a motion trajectory. The wearable device may determine, based on the motion data or the motion trajectory, whether the user is in the stationary state.

In this way, when measuring blood pressure, the wearable device may determine whether a user status meets the blood pressure measurement condition. When the blood pressure measurement condition is met, the wearable device may directly save a blood pressure measurement result. When the blood pressure measurement condition is not met, the wearable device needs to correct a blood pressure measurement result, and then save a corrected blood pressure measurement result, to improve accuracy of blood pressure measurement.

With reference to the first aspect, in a possible implementation, the method further includes: The wearable device obtains historical blood pressure measurement data, where the historical blood pressure measurement data includes a historical blood pressure measurement record and a historical blood pressure measurement result. The wearable device updates the blood pressure measurement scheme based on the historical blood pressure measurement data, and generates and saves a second updated blood pressure measurement scheme. The wearable device obtains a blood pressure measurement value of the user within one blood pressure measurement cycle based on the second updated blood pressure measurement scheme.

The historical blood pressure measurement record includes but is not limited to the historical blood pressure measurement record and the historical blood pressure measurement result. The historical blood pressure measurement record may include but is not limited to: a start moment of historical blood pressure measurement, an end moment of historical blood pressure measurement, a duration of historical blood pressure measurement, a quantity of times of blood pressure measurement within the duration of historical blood pressure measurement, a time interval between two adjacent times of blood pressure measurement within the duration of historical blood pressure measurement, a total quantity of times of historical blood pressure measurement, and the like.

In this way, the wearable device may periodically or aperiodically update the blood pressure measurement scheme based on the historical data of the user, to improve accuracy of the blood pressure measurement scheme generated by the wearable device.

According to a second aspect, this application provides a wearable device. The wearable device includes a memory and a processor. The memory is coupled to the processor. The memory is configured to store a computer program. When the processor invokes the computer program, the wearable device is enabled to perform the method for generating a blood pressure measurement scheme according to any possible implementation of any one of the foregoing aspects.

According to a third aspect, this application provides a computer-readable storage medium, including instructions. When the instructions are run on a wearable device, the wearable device is enabled to perform the method for generating a blood pressure measurement scheme according to any possible implementation of any one of the foregoing aspects.

According to a fourth aspect, this application provides a chip system. The chip system includes one or more processors. The processor is configured to invoke computer instructions, to perform the method for generating a blood pressure measurement scheme according to any possible implementation of any one of the foregoing aspects.

According to a fifth aspect, this application provides a computer program product including instructions. When the computer program product is run on a wearable device, the wearable device is enabled to perform the method for generating a blood pressure measurement scheme according to any possible implementation of any one of the foregoing aspects.

For descriptions of beneficial effects of the second aspect to the fifth aspect, refer to the descriptions of the beneficial effects of the first aspect. Details are not described herein again in this application.

### BRIEF DESCRIPTION OF DRAWINGS

FIG. 1 is a diagram of a user wearing a wearable device 100;
FIG. 2 is a diagram of a composition structure of a wearable device 100;
FIG. 3A is an example diagram of a principle of an oscillometric method according to an embodiment of this application;
FIG. 3B is another example diagram of a principle of an oscillometric method according to an embodiment of this application;
FIG. 4A is a diagram of a hardware structure of a wearable device 100;
FIG. 4B is a diagram of a composition structure of an airbag, an air pump, and an air path communication component according to an embodiment of this application;
FIG. 5A to FIG. 5D are diagrams of manually setting a blood pressure measurement scheme by a user;
FIG. 6A and FIG. 6B are diagrams of recommending a blood pressure measurement scheme by a wearable device 100 to a user;
FIG. 7A and FIG. 7B are a schematic flowchart of a method for prompting, by a wearable device 100 based on a remaining battery level, a user to change a blood pressure measurement scheme;
FIG. 8A to FIG. 8D are diagrams of prompting, by a wearable device 100, a user to change a blood pressure measurement scheme;
FIG. 9 is a schematic flowchart of a method for measuring blood pressure of a user by a wearable device 100;
FIG. 10A to FIG. 10E are diagrams of displaying, by a wearable device 100, prompt information to prompt a user to measure blood pressure;
FIG. 11 is a schematic flowchart of another method for measuring blood pressure of a user by a wearable device 100;
FIG. 12 is a schematic flowchart of still another method for measuring blood pressure of a user by a wearable device 100;
FIG. 13A and FIG. 13B are diagrams of prompting, by an electronic device 200, a user to charge a wearable device 100 as soon as possible;
FIG. 13C and FIG. 13D are diagrams of prompting, by an electronic device 200, a user to wear a wearable device 100 as soon as possible;
FIG. 14A to FIG. 14D are diagrams of displaying, by a wearable device 100 or an electronic device 200, a blood pressure measurement result within one cycle; and
FIG. 15 is a schematic method flowchart of a method for generating a blood pressure measurement scheme according to this application.

### DESCRIPTION OF EMBODIMENTS

The following clearly describes the technical solutions in embodiments of this application in detail with reference to the accompanying drawings. In descriptions of embodiments of this application, "/" indicates or, unless otherwise specified. For example, A/B may indicate A or B. In this specification, "and/or" describes only an association relationship between associated objects, and indicates that three relationships may exist. For example, A and/or B may indicate the following three cases: Only A exists, both A and B exist, and only B exists. In addition, in descriptions of embodiments of this application, "a plurality of" means two or more than two.

The terms "first" and "second" used below are merely intended for description, and shall not be understood as an indication or implication of relative importance or an implicit indication of a quantity of indicated technical features. Therefore, a feature limited by "first" or "second" may explicitly or implicitly include one or more features. In descriptions of embodiments of this application, "a plurality of" means two or more than two, unless otherwise specified.

The term "user interface (user interface, UI)" in the following embodiments of this application is a medium interface for interaction and information exchange between an application or an operating system and a user. The user interface implements conversion between an internal form of information and a form acceptable to the user. The user interface is usually represented in a form of a graphical user interface (graphical user interface, GUI), and is a user interface that is related to a computer operation and that is displayed in a graphic manner. The user interface may be a visual interface element, for example, text, an icon, a button, a menu, a tab, a text box, a dialog box, a status bar, a navigation bar, or a widget, that is displayed on a display of an electronic device.

With development of electronic technologies, functions of wearable devices are continuously enhanced. For example, a wearable device like a band or a watch may provide a blood pressure measurement function, to help a user measure blood pressure of the user anytime and anywhere, to learn of a physical condition of the user. The user may wear the wearable device on a wrist, to conveniently monitor blood pressure of the user in real time.

The following describes a wearable device 100, provided in this application, that can be used to measure blood pressure.

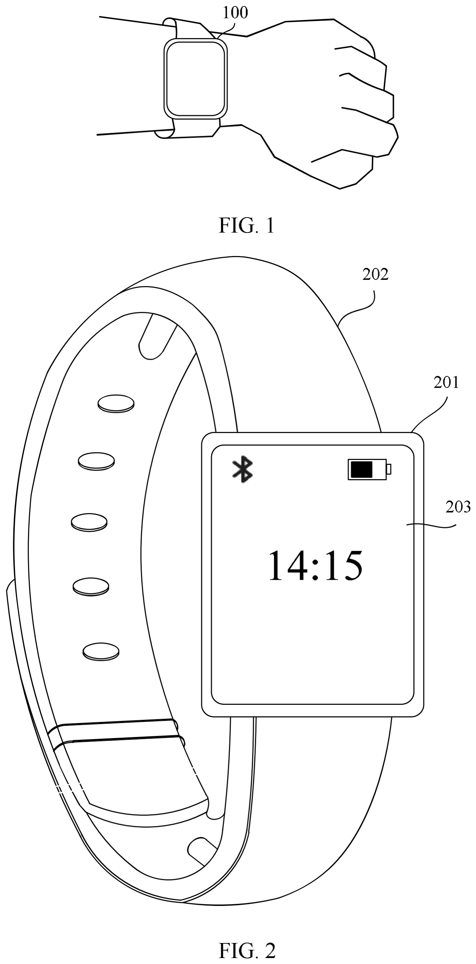

FIG. 1 is a diagram of a user wearing the wearable device 100.

As shown in FIG. 1, the user may wear the wearable device 100 on a wrist of the user.

FIG. 2 is a diagram of a composition structure of the wearable device 100.

As shown in FIG. 2, the wearable device 100 may include a watch body 201 and a wearable component 202.

The watch body 201 is equipped with a motion sensor, for example, a gyroscope sensor and an acceleration sensor. The motion sensor is configured to collect motion data, and determine, based on a motion status obtained through analysis based on the motion data, whether the user is in a sleep state.

The watch body 201 may include a display 203. The display 203 may be configured to display time, a battery level of the watch body 201, a Bluetooth identifier, a received message, motion data of the user, and other content. The display 203 may be configured to receive a tap operation of the user to turn on the display, enable or disable a sport mode, or the like. The display 203 may further record a quantity of moving steps and consumed calories of the user, and has basic functions such as an incoming call reminder and a message notification. In a possible implementation, the watch body 201 may establish a wireless communication connection to the wearable device 100 through Bluetooth. The watch body 201 may send the motion data of the user to the wearable device 100 to which the connection is established. In addition, when the wearable device 100 receives an incoming call or a message notification, the watch body 201 may receive an instruction from a mobile phone, to inform the user of the incoming call or the message notification.

The wearable component 202 is used for mounting the watch body 201. For example, the wearable component 202 may be a wristband strap, a watch strap, or another apparatus. The wearable component 202 is an apparatus that can attach the watch body 201 to the wrist of the user. The wearable device 100 is attached to the wrist of the user, so that an inertial sensor can collect motion data of the wrist of the user, to monitor motion of the wrist of the user and determine a posture of the user.

When the wearable device 100 starts to measure blood pressure, the wearable device 100 may control the wearable component 202 to shrink and then expand, to measure blood pressure of the user.

In some embodiments, a process of measuring blood pressure by the wearable device 100 may include: The wearable device 100 first inflates the wearable component 202 to temporarily block an upper-limb artery; then records, during slow deflation, a pressure value of the wearable component 202 and a pulse signal generated by a pulse; and finally determines blood pressure of the user based on the pressure value of the wearable component 202 and an amplitude or an envelope of the pulse signal. Blood flow causes lateral pressure to a blood vessel wall. A change in a magnitude of the lateral pressure causes slight vibration of the blood vessel wall. The pulse signal is a signal generated through the slight vibration of the blood vessel wall. Determining the blood pressure of the user based on the pressure value of the wearable component 202 and the amplitude or the envelope of the pulse signal is also referred to as an oscillometric method.

In another embodiment, a process of measuring blood pressure by the wearable device 100 may include: The wearable device 100 may gradually inflate the wearable component 202, so that an upper-limb artery changes from being gradually blocked to being completely blocked; record a pressure value of the wearable component 202 and a pulse signal generated by a pulse; then determine blood pressure of the user based on the pressure value of the wearable component 202 and an amplitude or an envelope of the pulse signal; and finally perform deflation. Blood flow causes lateral pressure to a blood vessel wall. A change in a magnitude of the lateral pressure causes slight vibration of the blood vessel wall. The pulse signal is a signal generated through the slight vibration of the blood vessel wall. Determining the blood pressure of the user based on the pressure value of the wearable component 202 and the amplitude or the envelope of the pulse signal is also referred to as an oscillometric method.

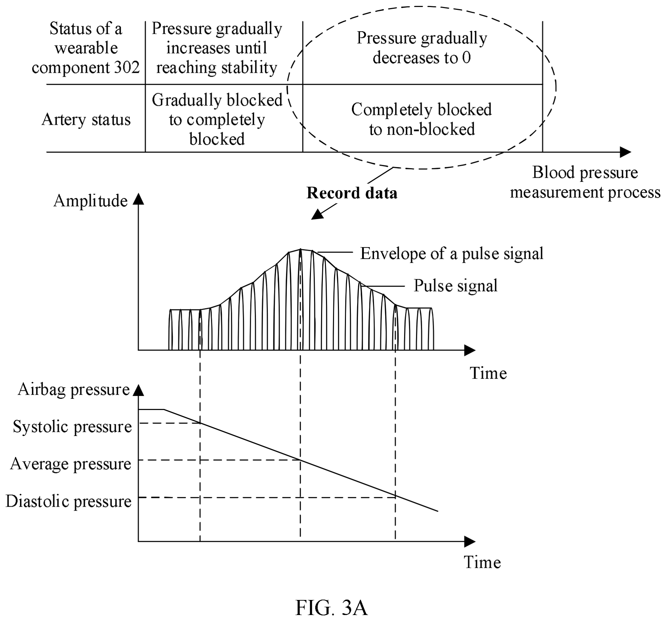

FIG. 3A is an example diagram of a principle of an oscillometric method according to an embodiment of this application.

As shown in FIG. 3A, in a process in which the wearable device 100 inflates the wearable component 202 to temporarily block the upper-limb artery, the wearable component 202 is in a state in which pressure gradually increases until reaching stability, and the artery in a state of being gradually blocked to being completely blocked. Then, during slow deflation, the wearable component 202 is in a state in which pressure gradually decreases to 0, and the artery is in a state of being completely blocked to being non-blocked. When the wearable component 202 is in the state in which the pressure gradually decreases to 0, a pressure value and a pulse signal of the wearable component 202 are recorded. When the pressure value of the wearable component 202 is greater than or equal to systolic pressure, the artery is blocked, and the pulse signal is a fine oscillation wave. When the pressure value of the wearable component 202 gradually decreases and is less than the systolic pressure and greater than average pressure, the artery gradually becomes non-blocked, and an amplitude of the pulse signal continuously increases. When the pressure value of the wearable component 202 is equal to the average pressure, the amplitude of the pulse signal reaches a maximum value. When the pressure value of the wearable component 202 continues to gradually decrease and is greater than diastolic pressure and less than the average pressure, the amplitude of the pulse signal gradually decreases. When the pressure value of the wearable component 202 is less than the diastolic pressure, the pulse signal is a fine oscillation wave. Therefore, the wearable device 100 may determine the systolic pressure and the diastolic pressure of the user based on the pressure value of the wearable component 202 and a change in the amplitude of the pulse signal. In a possible implementation, the pressure value and the pulse signal of the wearable component 202 may be determined by a built-in pressure sensor in the wearable device 100.

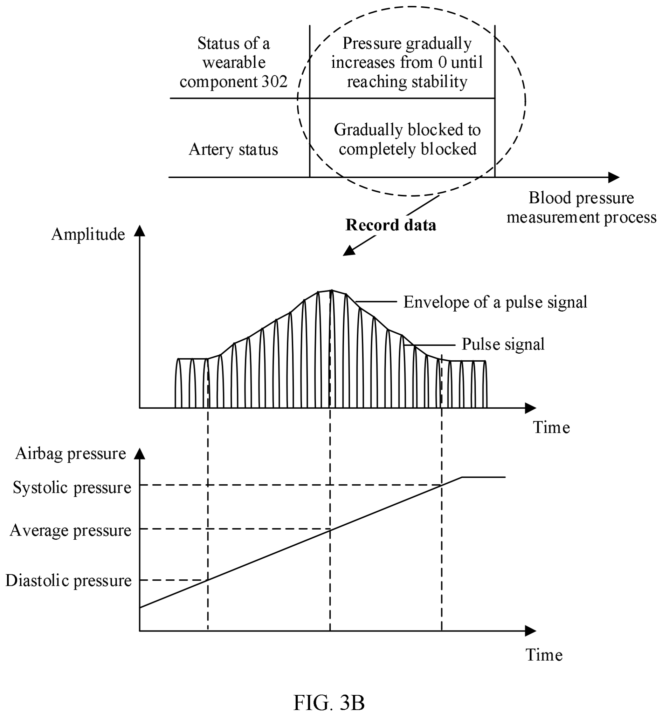

FIG. 3B is another example diagram of a principle of an oscillometric method according to an embodiment of this application.

As shown in FIG. 3B, in a process in which the wearable device 100 inflates the wearable component 202 to temporarily block the upper-limb artery, the wearable component 202 is in a state in which pressure gradually increases until reaching stability, and the artery in a state of being gradually blocked to being completely blocked. When the wearable component 202 is in the state in which the pressure gradually increases until reaching stability, a pressure value and a pulse signal of the wearable component 202 are recorded. When the pressure value of the wearable component 202 gradually increases and diastolic pressure is less than average pressure, the pulse signal is a fine oscillation wave. When the pressure value of the wearable component 202 continues to gradually increase and is greater than the diastolic pressure and less than the average pressure, an amplitude of the pulse signal gradually increases. When the pressure value of the wearable component 202 is equal to the average pressure, the amplitude of the pulse signal reaches a maximum value. When the pressure value of the wearable component 202 gradually increases and is greater than the average pressure and less than systolic pressure, the artery is gradually blocked, and the amplitude of the pulse signal continuously decreases. When the pressure value of the wearable component 202 is greater than or equal to the systolic pressure, the artery is blocked, and the pulse signal is a fine oscillation wave. Therefore, the wearable device 100 may determine the systolic pressure and the diastolic pressure of the user based on the pressure value of the wearable component 202 and a change in the amplitude of the pulse signal. In a possible implementation, the pressure value and the pulse signal of the wearable component 202 may be determined by a built-in pressure sensor in the wearable device 100.

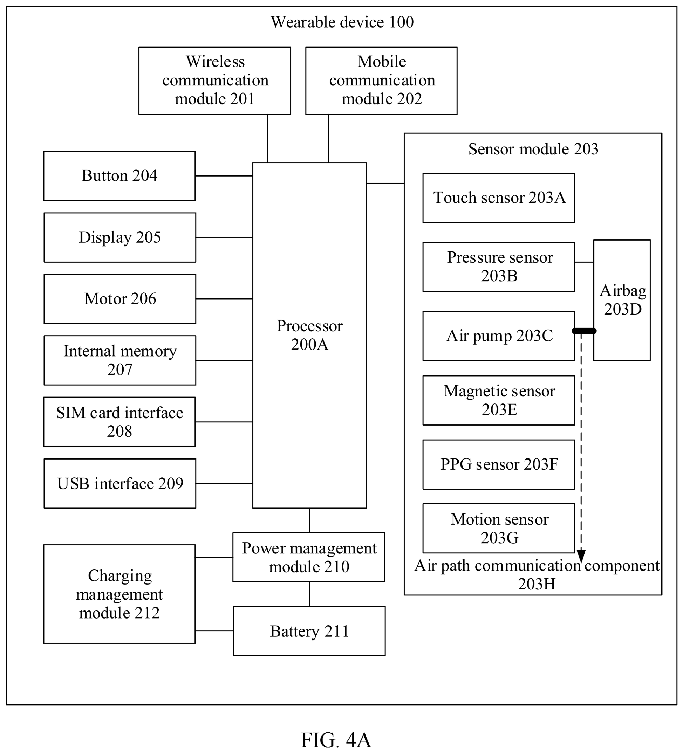

FIG. 4A is a diagram of a hardware structure of a wearable device 100.

As shown in FIG. 4A, the wearable device may be a band, a watch, or another wearable device; or the wearable device 100 may be a non-wearable device like a wall-mounted blood pressure monitor. A specific type of the wearable device is not particularly limited in this embodiment of this application. In this embodiment of this application, only an example in which the wearable device 100 is a watch is used for description.

The wearable device 100 may include a processor 200A, a wireless communication module 201, a mobile communication module 202, a sensor module 203, a button 204, a display 205, a motor 206, an internal memory 207, a SIM card interface 208, a USB interface 209, a power management module 210, a battery 211, and a charging management module 212. The sensor module 203 may include a touch sensor 203A, a pressure sensor 203B, an air pump 203C, an airbag 203D, a magnetic sensor 203E, a photoplethysmography (photoplethysmography, PPG) sensor 203F, a motion sensor 203G, and an air path communication component 203H. A function of the airbag 203D is similar to a function of the wearable component 202.

It can be understood that the structure shown in this embodiment of the present invention does not constitute a specific limitation on the wearable device. In some other embodiments of this application, the wearable device may include more or fewer components than those shown in the figure, or some components may be combined, or some components may be split, or the components may be arranged differently. The components shown in the figure may be implemented by using hardware, software, or a combination of software and hardware.

The processor 200A may include one or more processing units. For example, the processor 200A may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

In some embodiments, the processor 200A may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

In some embodiments, the processor 200A may alternatively be a micro processing unit (microcontroller unit, MCU).

The I2C interface is a two-way synchronous serial bus, and includes a serial data line (serial data line, SDA) and a serial clock line (serial clock line, SCL). In some embodiments, the processor 200A may include a plurality of groups of I2C buses. The processor 200A may be coupled to the touch sensor 203A, the power management module 210, and the like separately through different I2C bus interfaces. For example, the processor 200A may be coupled to the touch sensor 203A through the I2C interface, so that the processor 200A communicates with the touch sensor 203A through the I2C bus interface, to implement a touch function of the wearable device.

The I2S interface may be used for audio communication. The PCM interface may also be used for audio communication, and sampling, quantization, and encoding of an analog signal. The UART interface is a universal serial data bus, and is used for asynchronous communication. The bus may be a two-way communication bus. The bus converts to-be-transmitted data between serial communication and parallel communication. In some embodiments, the UART interface is usually configured to connect the processor 200A to the wireless communication module 201. For example, the processor 200A communicates with a Bluetooth module in the wireless communication module 201 through the UART interface, to implement a Bluetooth function.

The MIPI interface may be configured to connect the processor 200A to a peripheral component like the display 205. The MIPI interface includes a camera serial interface (camera serial interface, CSI), a display serial interface (display serial interface, DSI), and the like. The processor 200A communicates with the display 205 through the DSI interface, to implement a display function of the wearable device.

The GPIO interface may be configured by software. The GPIO interface may be configured as a control signal or a data signal. The USB interface 209 is an interface that conforms to a USB standard specification, and may be specifically a mini USB interface, a micro USB interface, a USB Type-C interface, or the like. The USB interface 209 may be configured to connect to a charger to charge the wearable device, or may be configured to transmit data between the wearable device and a peripheral device.

It can be understood that an interface connection relationship between the modules in this embodiment of the present invention is merely an example for description, and does not constitute a limitation on a structure of the wearable device. In some other embodiments of this application, the wearable device may alternatively use an interface connection mode different from that in the foregoing embodiment, or use a combination of a plurality of interface connection modes.

The charging management module 212 is configured to receive a charging input from a charger. The charger may be a wireless charger or a wired charger. In some embodiments of wired charging, the charging management module 212 may receive a charging input from a wired charger through the USB interface 209. In some embodiments of wireless charging, the charging management module 212 may receive a wireless charging input through a wireless charging coil of the wearable device. When charging the battery 211, the charging management module 212 may further supply power to the wearable device through the power management module 210.

The power management module 210 is configured to connect to the battery 211, the charging management module 212, and the processor 200A. The power management module 210 receives an input from the battery 211 and/or the charging management module 212, and supplies power to the processor 200A, the internal memory 207, the display 205, the wireless communication module 201, and the like. The power management module 210 may be further configured to monitor parameters such as a battery capacity, a quantity of battery cycles, and a battery health status (electric leakage or impedance). In some other embodiments, the power management module 210 may alternatively be disposed in the processor 200A. In some other embodiments, the power management module 210 and the charging management module 212 may alternatively be disposed in a same component.

A wireless communication function of the wearable device may be implemented by the mobile communication module 202, the wireless communication module 201, the modem processor, the baseband processor, and the like.

The mobile communication module 202 may provide a solution applied to the wearable device for wireless communication such as 2G/3G/4G/5G. The mobile communication module 202 may include at least one filter, a switch, a power amplifier, a low noise amplifier (low noise amplifier, LNA), and the like. The mobile communication module 202 may receive an electromagnetic wave through the antenna, perform processing such as filtering or amplification on the received electromagnetic wave, and transmit a processed electromagnetic wave to the modem processor for demodulation. In some embodiments, at least some functional modules of the mobile communication module 202 may be disposed in the processor 200A. In some embodiments, at least some functional modules of the mobile communication module 202 may be disposed in a same component as at least some modules of the processor 200A.

The wireless communication module 201 may provide a solution applied to the wearable device for wireless communication such as a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, or an infrared (infrared, IR) technology. The wireless communication module 201 may be one or more components integrating at least one communications processor module. The wireless communication module 201 receives an electromagnetic wave through the antenna, performs frequency modulation and filtering on an electromagnetic wave signal, and sends a processed signal to the processor 200A. The wireless communication module 201 may further receive a to-be-sent signal from the processor 200A, perform frequency modulation and amplification on the signal, and convert a processed signal into an electromagnetic wave for radiation through the antenna.

The button 204 includes a power button, a volume button, and the like. The button 204 may be a mechanical button or a touch button. The wearable device may receive an input on the button, and generate a button signal input related to a user setting and function control of the wearable device.

The display 205 is configured to display an image, a video, or the like. The display 205 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flex light-emitting diode, FLED), a quantum dot light-emitting diode (quantum dot light-emitting diode, QLED), or the like. In some embodiments, the wearable device may include one or N displays 205, where N is a positive integer greater than 1.

The motor 206 may generate a vibration prompt. The motor 206 may be configured to produce an incoming call vibration prompt or a touch vibration feedback. For example, touch operations performed on different applications (for example, photographing and audio playing) may correspond to different vibration feedback effects. The motor 206 may also correspond to different vibration feedback effects for touch operations performed on different areas of the display 205.

The internal memory 207 may include one or more random access memories (random access memory, RAM) and one or more non-volatile memories (non-volatile memory, NVM).

The random access memory may include a static random access memory (static random access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, DDR SDRAM, for example, a fifth-generation DDR SDRAM is usually referred to as a DDR5 SDRAM), and the like.

The non-volatile memory may include a magnetic disk storage device and a flash memory (flash memory). The flash memory may include a NOR flash, a NAND flash, a 3D NAND flash, and the like through division according to an operation principle; may include a single-level cell (single-level cell, SLC), a multi-level cell (multi-level cell, MLC), a triple-level cell (triple-level cell, TLC), a quad-level cell (quad-level cell, QLC), and the like through division based on a potential order of a storage unit; or may include a universal flash storage (English: universal flash storage, UFS), an embedded multimedia card (embedded multimedia card, eMMC), and the like through division based on storage specifications. The processor 200A may directly perform a read or write operation on the random access memory. The random access memory may be configured to store executable programs (for example, machine instructions) of an operating system or another running program, and may be further configured to store data of a user and an application, and the like. The non-volatile memory may also store an executable program, data of the user and an application, and the like, which may be pre-loaded to the random access memory for the processor 200A to directly perform a read or write operation.

The SIM card interface 208 is used for connecting a SIM card. The SIM card may be inserted into the SIM card interface 208 or removed from the SIM card interface 208, to implement contact with or separation from the wearable device. The wearable device may support one or N SIM card interfaces, where N is a positive integer greater than 1. The SIM card interface 208 may support a nano-SIM card, a micro-SIM card, a SIM card, and the like. A plurality of cards may be inserted in a same SIM card interface 208 at the same time. The plurality of cards may be of a same type or different types. The SIM card interface 208 is also compatible with different types of SIM cards. The SIM card interface 208 is also compatible with an external memory card. The wearable device interacts with a network through the SIM card, to implement functions such as calling and data communication. In some embodiments, an eSIM, namely, an embedded SIM card, is used for the wearable device. The eSIM card may be embedded into the wearable device, and cannot be separated from the wearable device.

In some embodiments, the wearable device 100 may alternatively not include the SIM card interface 208.

The touch sensor 203A is also referred to as a "touch device". The touch sensor 203A may be disposed in the display 205. The touch sensor 203A and the display 205 constitute a touchscreen, which is also referred to as a "touch screen". The touch sensor 203A is configured to detect a touch operation performed on or near the touch sensor. The touch sensor may transmit the detected touch operation to the application processor to determine a type of a touch event. The display 205 may provide a visual output related to the touch operation. In some other embodiments, the touch sensor 203A may alternatively be disposed on a surface of the wearable device at a position different from a position of the display 205.

The pressure sensor 203B is configured to measure pressure. In some embodiments of this application, the wearable device measures pressure in the airbag 203D through the pressure sensor 203B. In some embodiments of this application, some components of the pressure sensor 203B are located in the airbag 203D, and are configured to sense pressure in the airbag 203D.

The air pump 203C is configured to perform inflation and deflation. In some embodiments of this application, the wearable device inflates the airbag 203D through the air pump 203C, where the air pump 203C is connected to the airbag 203D through the air path communication component 203H. The airbag 203D is configured to press against a blood vessel of the user.

The magnetic sensor 203E includes a Hall effect sensor. In some embodiments of this application, the wearable device may determine, through the magnetic sensor 203F, whether the airbag 203D on the wearable device is removed. For example, the airbag 203D or a watch strap connected to the airbag 203D may be equipped with a magnet. The wearable device may determine, through the magnetic sensor, a magnetic flux generated by the airbag 203D or the magnet on the airbag 203D, to determine whether the airbag 203D on the wearable device is removed.

The PPG sensor 203F is configured to obtain health data of the user based on a PPG signal collected by the PPG sensor 203F. The health data of the user includes but is not limited to a heart rate, blood oxygen, a respiratory rate, blood oxygen saturation (SaO₂), and the like.

The motion sensor 203G includes but is not limited to an acceleration sensor and an angular velocity sensor. The motion sensor 203G may be configured to collect motion data, determine a motion status of the user based on the motion data, and then determine, based on the motion status of the user, whether the user is in a sleep state.

It should be noted that the air path communication component 203H may be an independent component, or the air path communication component 203H may be an air path formed by combining other hardware modules, or the air path communication component 203H may be a part of another component, for example, a part of the air pump 203C, or a part of the airbag 203D.

It should be noted that the sensor module 203 may further include an acceleration sensor, an infrared sensor, and the like.

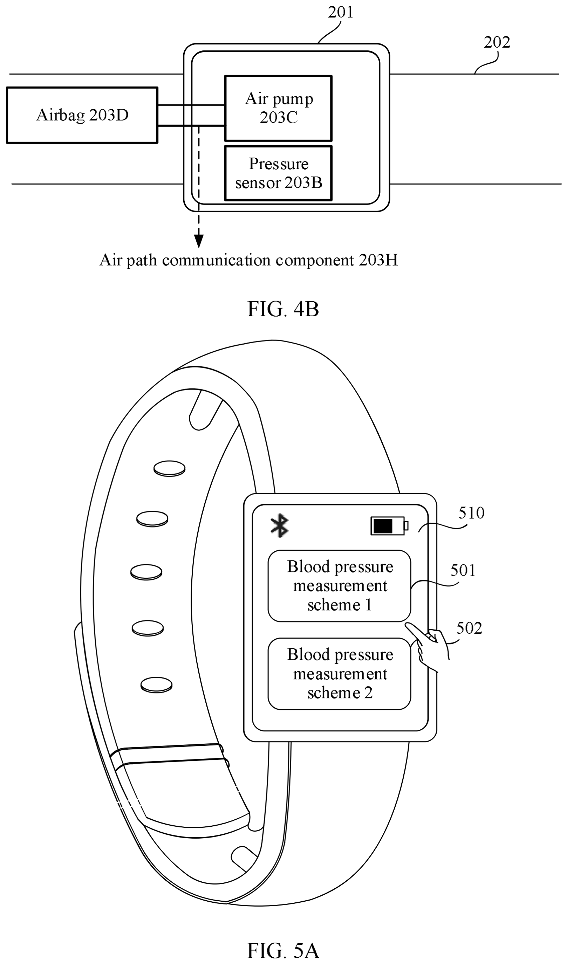

As shown in FIG. 4B, when the wearable device 100 is a watch, the airbag 203D is attached to a side, close to a body, of the wearable component 202. The air pump 203C is connected to the airbag 203D through the air path communication component 203H. The airbag 203D may be attached only to one side of the wearable component 202, and the side of the wearable component 202 may be located above an artery position on a wrist of the user, for example, above a radial artery position.

The air pump 203C may be located in a watch body of the smartwatch. The airbag 203D may be connected to a buckle of a watch strap, and the airbag 203D is connected to a watch face through an air vent cover. Correspondingly, the airbag 203D may be separated from the watch strap, or may be separated from the watch face.

Currently, a user may set a blood pressure measurement scheme on the wearable device 100.

The blood pressure measurement scheme may include one or more blood pressure measurement policies. The blood pressure measurement policy may include but is not limited to: a start moment of blood pressure measurement, an end moment of blood pressure measurement, a duration of blood pressure measurement, a quantity of times of blood pressure measurement within the duration of blood pressure measurement, a time interval between two adjacent times of blood pressure measurement within the duration of blood pressure measurement, a total quantity of times of blood pressure measurement, and the like.

The start moment of blood pressure measurement may be a moment of a day at which measurement on blood pressure of the user is started. For example, the start moment of blood pressure measurement may be 9:00 a.m. or 10:00 p.m.

The end moment of blood pressure measurement may be a moment of a day at which measurement on blood pressure of the user is stopped. For example, the end moment of blood pressure measurement may be 12:00 noon or 6:00 a.m.

The duration of blood pressure measurement may be a time period, between two moments of a day, within which blood pressure of the user is measured. A duration of blood pressure measurement within one day may include one or more time periods. For example, the duration of blood pressure measurement may be 9:00 a.m. to 12:00 noon, and/or 10:00 p.m. to 6:00 a.m. of a next day.

Blood pressure measurement frequency within the duration of blood pressure measurement may be a total quantity of times of blood pressure measurement within one duration of blood pressure measurement. For example, when the duration of blood pressure measurement is 9:00 a.m. to 12:00 noon, the blood pressure measurement frequency is 6 times; or when the duration of blood pressure measurement is 10:00 p.m. to 6:00 a.m. of a next day, the blood pressure measurement frequency is 30 times.

The time interval between two adjacent times of blood pressure measurement within the duration of blood pressure measurement may be a time difference between two adj acent blood pressure measurement moments within one duration of blood pressure measurement. The time difference between two adjacent blood pressure measurement moments within one duration of blood pressure measurement may remain the same or vary. For example, when the duration of blood pressure measurement is 9:00 a.m. to 12:00 noon, the time difference between two adjacent blood pressure measurement moments may be 30 minutes.

The total quantity of times of blood pressure measurement may be a total quantity of times of blood pressure measurement within 24 hours of one day from 0:00 to 12:00 midnight. For example, the total quantity of times of blood pressure measurement within one day may be 36 times.

In addition to the foregoing blood pressure measurement policies, the blood pressure measurement scheme may further include another blood pressure measurement policy. This is not limited in this application.

**FIG. 5A to FIG. 5D** **are diagrams of manually setting a blood pressure measurement scheme by a user.**

For example, a wearable device 100 may display a user interface 510 shown in FIG. 5A. An option 501 and an option 502 are displayed on the user interface 510. The user may manually set a blood pressure measurement scheme by using the option 501 and the option 502.

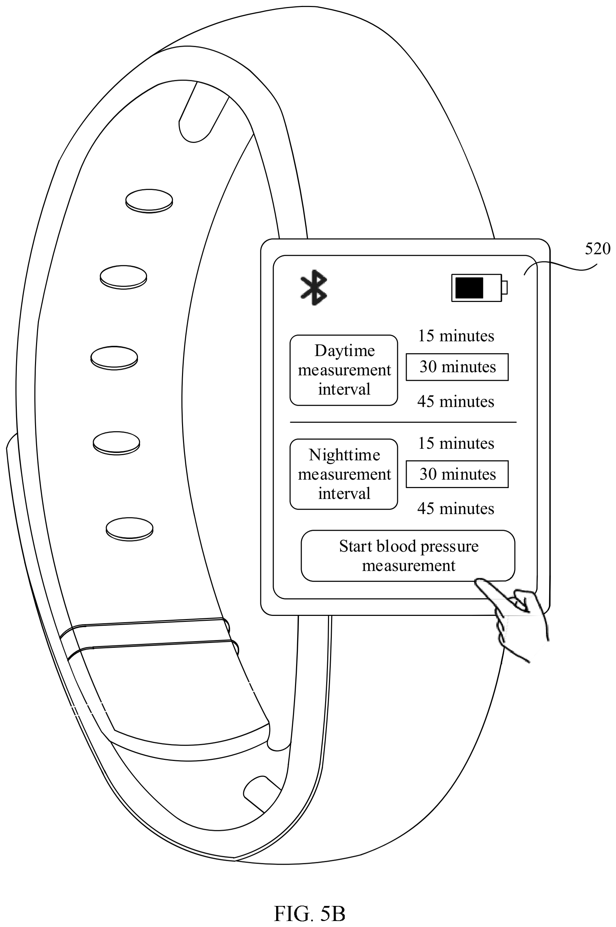

For example, as shown in FIG. 5A, the wearable device 100 may receive an input operation (for example, tapping) of the user for the option 501 on the user interface 510, and in response to the input operation of the user, the wearable device 100 may display a user interface 520 shown in FIG. 5B. Some blood pressure measurement policies of a blood pressure measurement scheme are displayed on the user interface 520. For example, the user may manually set a time interval between two adjacent times of blood pressure measurement at daytime and a time interval between two adj acent times of blood pressure measurement at nighttime. For example, the time interval between two adjacent times of blood pressure measurement at daytime that is set by the user is 30 minutes, and the time interval between two adjacent times of blood pressure measurement at nighttime that is set by the user is also 30 minutes.

In addition to the time interval between blood pressure measurement, the wearable device 100 may further receive a user operation to set another blood pressure measurement policy. This is not limited in this application.

Then the wearable device 100 may receive an input operation (for example, tapping) of the user for a Start blood pressure measurement option on the user interface 520. In response to the input operation of the user, the wearable device 100 may save the blood pressure measurement scheme that is set by the user, and start to measure blood pressure of the user when a condition of the blood pressure measurement scheme is met.

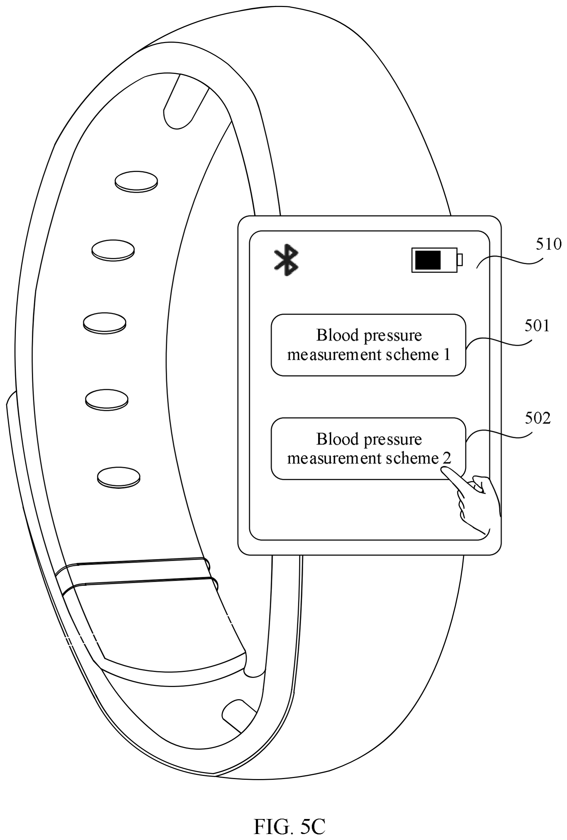

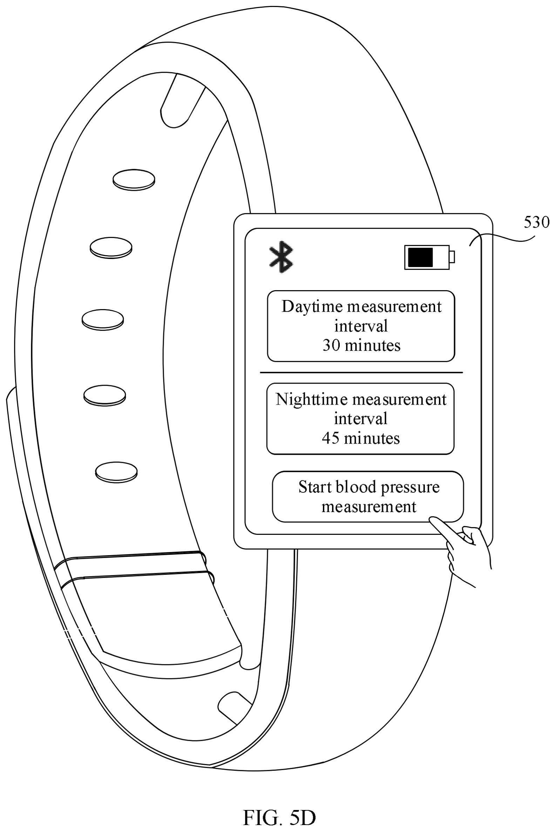

For another example, as shown in FIG. 5C, the wearable device 100 may receive an input operation (for example, tapping) of the user for the option 502 on the user interface 510, and in response to the input operation of the user, the wearable device 100 may display a user interface 530 shown in FIG. 5D. Some blood pressure measurement policies of a blood pressure measurement scheme are displayed on the user interface 530. The blood pressure measurement scheme may be preset. For example, the preset blood pressure measurement scheme includes a time interval between two adjacent times of blood pressure measurement at daytime and a time interval between two adj acent times of blood pressure measurement at nighttime. For example, the time interval between two adj acent times of blood pressure measurement at daytime is 30 minutes, and the time interval between two adjacent times of blood pressure measurement at nighttime is also 30 minutes.

Then the wearable device 100 may receive an input operation (for example, tapping) of the user for a Start blood pressure measurement option on the user interface 530. In response to the input operation of the user, the wearable device 100 may save the preset blood pressure measurement scheme, and start to measure blood pressure of the user when a condition of the preset blood pressure measurement scheme is met.

It should be noted that FIG. 5A to FIG. 5D are merely diagrams of manually creating a blood pressure measurement scheme, and the user may alternatively manually create a blood pressure measurement scheme in another implementation. This is not limited in this application.

It can be learned from FIG. 5A to FIG. 5D that a blood pressure measurement scheme needs to be manually created by a user or manually set to take effect. A user operation is complex, and the blood pressure measurement scheme manually created by the user or manually set to take effect does not necessarily meet a user requirement, leading to poor user experience.

In view of this, this application provides a method for generating a blood pressure measurement scheme. A wearable device 100 may automatically generate a blood pressure measurement scheme based on one or more of the following information: user information, physiological data, a device status, and the like. A user does not need to manually create a blood pressure measurement scheme, so that a user operation is simplified.

The blood pressure measurement scheme may include one or more blood pressure measurement policies. The blood pressure measurement policy may include but is not limited to: a start moment of blood pressure measurement, an end moment of blood pressure measurement, a duration of blood pressure measurement, a quantity of times of blood pressure measurement within the duration of blood pressure measurement, a time interval between two adjacent times of blood pressure measurement within the duration of blood pressure measurement, a total quantity of times of blood pressure measurement, and the like.

A cycle of the blood pressure measurement scheme may be one day (24 hours), or may be another cycle. This is not limited in this application.

The user information includes but is not limited to one or more of the following information: a gender, an age, a height, a weight, a smoking status, an alcohol consumption status, medical history, a medication status, lifestyle habits, and the like.

The physiological data includes but is not limited to one or more of the following: a heart rate, blood pressure, arteriosclerosis, blood glucose, and the like.

The device status includes but is not limited to one or more of the following: a remaining battery level, device temperature, and the like.

The wearable device 100 may determine a hypertension risk of the user based on the user information, the physiological data, the device status, and the like, and obtain the blood pressure measurement scheme based on the hypertension risk of the user. Different blood pressure measurement schemes are obtained for different hypertension risks.

Optionally, the blood pressure measurement scheme may be a blood pressure measurement scheme within one day (24 hours).

Optionally, different users have different user information, physiological data, and device statuses, and the wearable device 100 obtains different blood pressure measurement schemes.

For example, when it is determined that the hypertension risk of the user is a high risk, a measurement mode of the blood pressure measurement scheme obtained by the wearable device 100 may be a high-frequency measurement mode; or
when it is determined that the hypertension risk of the user is a low risk, a measurement mode of the blood pressure measurement scheme obtained by the wearable device 100 may be a low-frequency measurement mode.

A total quantity of times of blood pressure measurement in the high-frequency measurement mode is greater than a total quantity of times of blood pressure measurement in the low-frequency measurement mode.

Optionally, the total quantity of times of blood pressure measurement may be a total quantity of times of blood pressure measurement within one day (24 hours).

In another embodiment, the high-frequency measurement mode and the low-frequency measurement mode may alternatively be measurement modes within a specific time period.

For example, when determining that the hypertension risk of the user is the high risk only within a first time period, the wearable device 100 may measure blood pressure of the user in the high-frequency measurement mode only within the first time period, and measure blood pressure of the user in the low-frequency measurement mode within a non-first time period.

For example, when determining that the hypertension risk of the user is the high risk only at 8:00 a.m. to 12:00 noon and the hypertension risk of the user is the low risk at 0:00 to 8:00 a.m. and at 12:00 noon to 12:00 midnight, the wearable device 100 may measure blood pressure of the user in the high-frequency measurement mode only at 8:00 a.m. to 12:00 noon, and measure blood pressure of the user in the low-frequency measurement mode at 0:00 to 8:00 a.m. and at 12:00 noon to 12:00 midnight.

In some embodiments, the wearable device 100 may further divide the high risk of the hypertension risk into different high risk levels, and enable different measurement modes based on different high risk levels.

For example, the high risk may be further divided into a first-level risk and a second-level risk, and the first-level risk is higher than the second-level risk. In addition to the two levels, the high risk may alternatively be divided into more other levels. This is not limited in this application.

When determining that the hypertension risk of the user is the first-level risk within a first time period and the hypertension risk is the second-level risk within a second time period, the wearable device 100 may measure blood pressure of the user in a first high-frequency measurement mode within the first time period, and measure blood pressure of the user in a second high-frequency measurement mode within the second time period. A total quantity of times of blood pressure measurement in the first high-frequency measurement mode is greater than a total quantity of times of blood pressure measurement in the second high-frequency measurement mode.

For example, when determining that the hypertension risk of the user is the first-level risk at 8:00 a.m. to 10:00 a.m., the hypertension risk of the user is the second-level risk at 10:00 a.m. to 12:00 noon, and the hypertension risk of the user is the low risk at 0:00 to 8:00 a.m. and at 12:00 noon to 12:00 midnight, the wearable device 100 may measure blood pressure of the user in the first high-frequency measurement mode at 8:00 a.m. and 10:00 a.m., measure blood pressure of the user in the second high-frequency measurement mode at 10:00 a.m. to 12:00 noon, and measure blood pressure of the user in the low-frequency measurement mode at 0:00 to 8:00 a.m. and at 12:00 noon to 12:00 midnight.

In some embodiments, the wearable device 100 may determine the start moment of blood pressure measurement based on the lifestyle habits of the user.

The wearable device 100 may determine the lifestyle habits of the user based on collected user data, for example, historical travel data and historical passenger records of the user; and then determine the start moment of blood pressure measurement based on the lifestyle habits of the user, to avoid disturbing daily activities of the user.

For example, the wearable device 100 may determine, based on the collected user data, that the user is on the way to work at 7:00 a.m. to 8:30 a.m. In this case, the start moment of blood pressure measurement in the blood pressure measurement scheme determined by the wearable device 100 may be beyond the period of 7:00 a.m. to 8:30 a.m.

Optionally, after the wearable device 100 determines the blood pressure measurement scheme, the wearable device 100 may prompt the user to view detailed content of the blood pressure measurement scheme and determine whether to save and use the blood pressure measurement scheme.

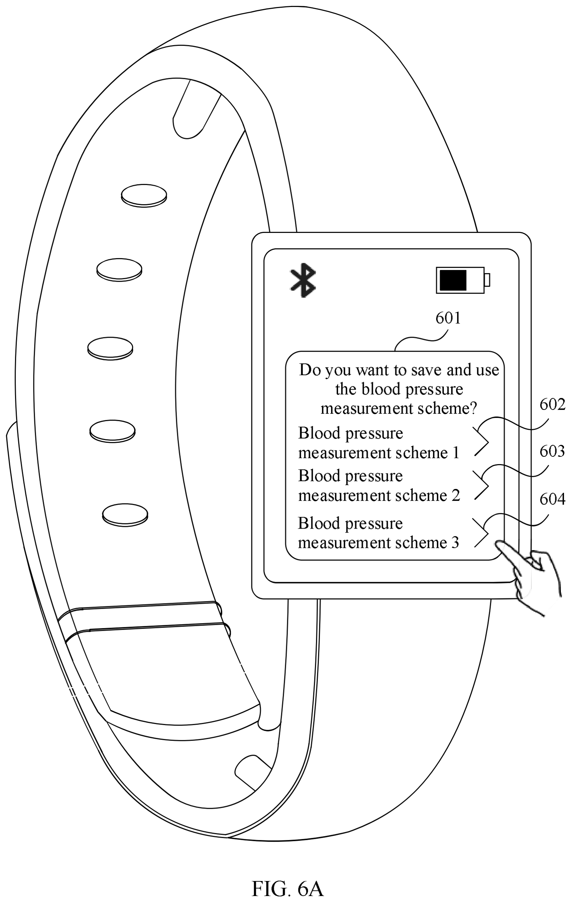

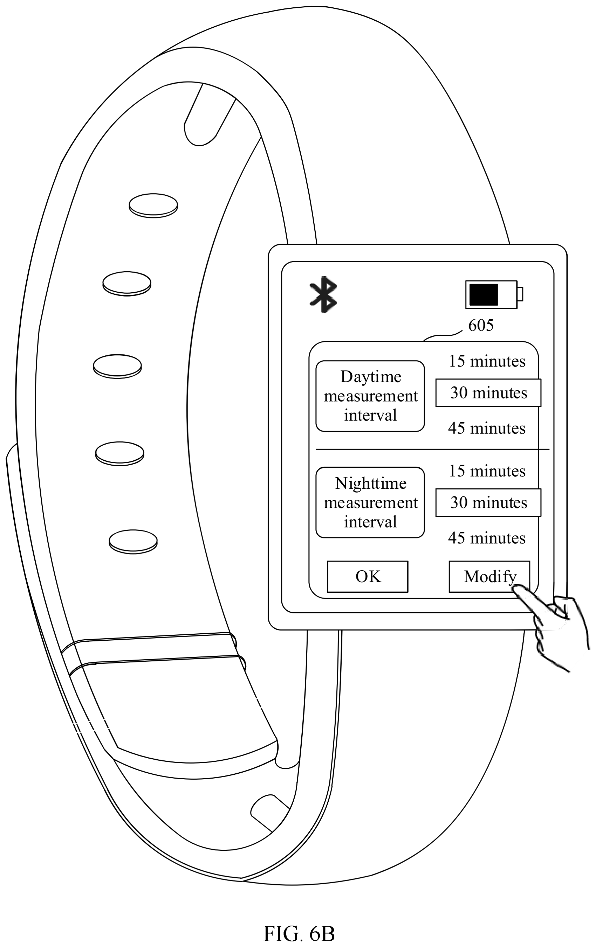

FIG. 6A and FIG. 6B are diagrams of recommending a blood pressure measurement scheme by a wearable device 100 to a user.

After the wearable device 100 determines a blood pressure measurement scheme, the wearable device 100 may prompt the user to view detailed content of the blood pressure measurement scheme, to prompt the user whether to save and use the blood pressure measurement scheme.

As shown in FIG. 6A, the wearable device 100 may display a prompt bar 601, and the prompt bar 601 includes prompt information: "Do you want to save and use the blood pressure measurement scheme?" The prompt bar 601 is used to prompt the user to view the detailed content of the blood pressure measurement scheme.

The prompt bar 601 includes an option 602, an option 603, and an option 604. The wearable device 100 may receive an input operation (for example, tapping) of the user for the option 602, and in response to the input operation of the user, the wearable device 100 may display detailed content of a blood pressure measurement scheme 1. Alternatively, the wearable device 100 may receive an input operation (for example, tapping) of the user for the option 603, and in response to the input operation of the user, the wearable device 100 may display detailed content of a blood pressure measurement scheme 2. Alternatively, the electrical wearable device 100 may receive an input operation (for example, tapping) of the user for the option 604, and in response to the input operation of the user, the wearable device 100 may display detailed content of a blood pressure measurement scheme 3.

For example, as shown in FIG. 6A, the wearable device 100 may receive an input operation (for example, tapping) of the user for the option 604 in the prompt bar 601, and in response to the input operation of the user, the wearable device 100 may display a prompt bar 605 shown in FIG. 6B, where the detailed content of the blood pressure measurement scheme 3 is displayed in the prompt bar 605. For example, the detailed content of the blood pressure measurement scheme 3 may include: A time interval between two adj acent times of blood pressure measurement at daytime is 30 minutes, and a time interval between two adjacent times of blood pressure measurement at nighttime is also 30 minutes. The user may view the detailed content of the blood pressure measurement scheme 3 in the prompt bar 605. The prompt bar 605 further includes an OK option and a Modify option. The user may save the blood pressure measurement scheme 3 by using the OK option. Alternatively, the user may modify the blood pressure measurement scheme 3 by using the Modify option, and then save a modified blood pressure measurement scheme 3.

**A wearable device 100 prompts, based on a remaining battery level, a user to change a blood pressure measurement scheme.**

In some embodiments, after the wearable device 100 determines and saves a blood pressure measurement scheme and before the wearable device 100 uses the blood pressure measurement scheme, the wearable device 100 may determine a minimum battery level needed by the blood pressure measurement scheme, and compare the minimum battery level needed by the blood pressure measurement scheme with a remaining battery level of the wearable device 100, to determine whether the saved blood pressure measurement scheme needs to be changed, to avoid a case in which the wearable device 100 is powered off due to an excessively low battery level and cannot measure blood pressure.

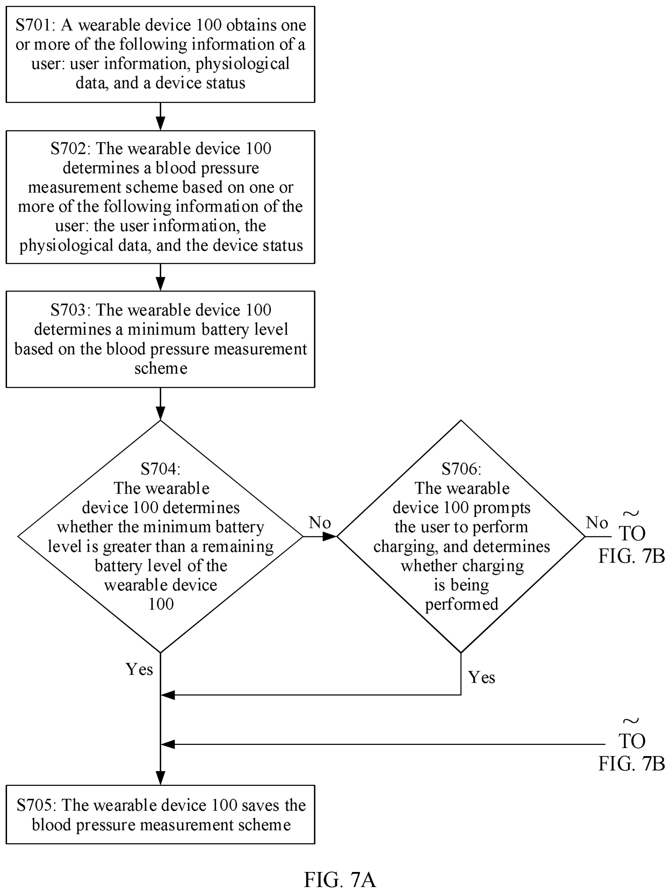

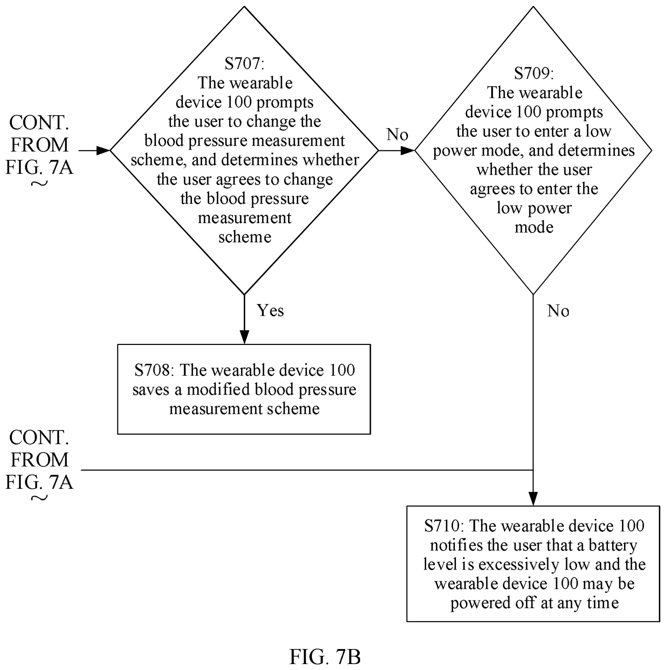

**FIG. 7A** **and** **FIG. 7B** **are a schematic flowchart of a method for prompting, by a wearable device 100 based on a remaining battery level, a user to change a blood pressure measurement scheme.**

S701: The wearable device 100 obtains one or more of the following information of the user: user information, physiological data, and a device status.

The user information includes but is not limited to one or more of the following information: a gender, an age, a height, a weight, a smoking status, an alcohol consumption status, medical history, a medication status, and the like.

The physiological data includes but is not limited to one or more of the following: a heart rate, blood pressure, arteriosclerosis, blood glucose, and the like.

The device status includes but is not limited to one or more of the following: a remaining battery level, device temperature, and the like.

The wearable device 100 may determine a hypertension risk of the user based on the user information, the physiological data, the device status, and the like, and obtain a blood pressure measurement scheme based on the hypertension risk of the user. Different blood pressure measurement schemes are obtained for different hypertension risks.

S702: The wearable device 100 determines a blood pressure measurement scheme based on one or more of the following information of the user: the user information, the physiological data, and the device status.

The blood pressure measurement scheme may include one or more blood pressure measurement policies. The blood pressure measurement policy may include but is not limited to: a start moment of blood pressure measurement, an end moment of blood pressure measurement, a duration of blood pressure measurement, a quantity of times of blood pressure measurement within the duration of blood pressure measurement, a time interval between two adjacent times of blood pressure measurement within the duration of blood pressure measurement, a total quantity of times of blood pressure measurement, and the like.

Optionally, the blood pressure measurement scheme may be a blood pressure measurement scheme within one day (24 hours).

Optionally, different users have different user information, physiological data, and device statuses, and the wearable device 100 obtains different blood pressure measurement schemes.

For example, when it is determined that the hypertension risk of the user is a high risk, a measurement mode of the blood pressure measurement scheme obtained by the wearable device 100 may be a high-frequency measurement mode; or
when it is determined that the hypertension risk of the user is a low risk, a measurement mode of the blood pressure measurement scheme obtained by the wearable device 100 may be a low-frequency measurement mode.

A total quantity of times of blood pressure measurement in the high-frequency measurement mode is greater than a total quantity of times of blood pressure measurement in the low-frequency measurement mode.

In another embodiment, the blood pressure measurement scheme may alternatively be determined by another device (for example, a mobile phone or a server) that has established a communication connection to the wearable device 100 and then sent to the wearable device 100.

S703: The wearable device 100 determines a minimum battery level based on the blood pressure measurement scheme.

The minimum battery level may be a minimum battery level needed for completing a total quantity of times of blood pressure measurement in the blood pressure measurement scheme within one day.

After the wearable device 100 obtains the blood pressure measurement scheme, the wearable device 100 may estimate, based on the blood pressure measurement scheme, the minimum battery level needed for completing the total quantity of times of blood pressure measurement within one day, to meet a requirement of a blood pressure measurement scheme for starting high-frequency blood pressure measurement.

Optionally, the wearable device 100 may estimate, based on an average battery level needed for completing blood pressure measurement once and the total quantity of times of blood pressure measurement in the blood pressure measurement scheme, the minimum battery level needed for completing the total quantity of times of blood pressure measurement within one day.

S704: The wearable device 100 determines whether the minimum battery level is greater than the remaining battery level of the wearable device 100.

The wearable device 100 may obtain the remaining battery level of the wearable device 100, and determine whether the minimum battery level is greater than the remaining battery level of the wearable device 100.

When it is determined that the minimum battery level is greater than the remaining battery level of the wearable device 100, S705 is performed.

When it is determined that the minimum battery level is less than the remaining battery level of the wearable device 100, S706 is performed.

S705: The wearable device 100 saves the blood pressure measurement scheme.

When it is determined that the minimum battery level is greater than the remaining battery level of the wearable device 100, the remaining battery level of the wearable device 100 can provide the minimum battery level needed for completing the total quantity of times of blood pressure measurement in the blood pressure measurement scheme within one day, and the wearable device 100 saves the blood pressure measurement scheme.

In some embodiments, before the wearable device 100 saves the blood pressure measurement scheme, the wearable device 100 may display user interfaces shown in FIG. 6A and FIG. 6B, to prompt the user to view the blood pressure measurement scheme. Alternatively, the user may actively modify the blood pressure measurement scheme, and save a modified blood pressure measurement scheme.

S706: The wearable device 100 prompts the user to perform charging, and determines whether charging is being performed.

When it is determined that the minimum battery level is less than the remaining battery level of the wearable device 100, the remaining battery level of the wearable device 100 cannot provide the minimum battery level needed for completing the total quantity of times of blood pressure measurement in the blood pressure measurement scheme within one day, and the wearable device 100 may prompt the user to charge the wearable device 100.

When it is determined that charging is being performed, S705 is performed.

When it is determined that charging is not being performed, S707 is performed.

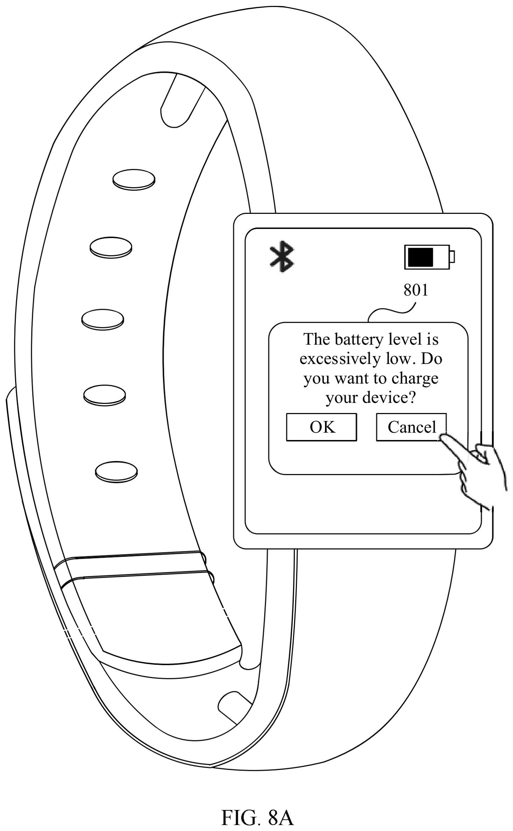

For example, when it is determined that the minimum battery level is less than the remaining battery level of the wearable device 100, the wearable device 100 may display prompt information 801 shown in FIG. 8A. The prompt information 801 includes text information: "The battery level is excessively low. Do you want to charge your device?" The prompt information 801 is used to prompt the user to charge the wearable device 100 as soon as possible. The prompt information 801 further includes an OK option and a Cancel option. The user may determine, by using the OK option, to charge the wearable device 100. Alternatively, the user may determine, by using the Cancel option, not to charge the wearable device 100.

S707: The wearable device 100 prompts the user to change the blood pressure measurement scheme, and determines whether the user agrees to change the blood pressure measurement scheme.

When determining that charging is not being performed, the wearable device 100 may prompt the user to change the blood pressure measurement scheme, and determine whether the user agrees to change the blood pressure measurement scheme.

Adjusting the blood pressure measurement scheme may be reducing the total quantity of times of blood pressure measurement in the blood pressure measurement scheme, to reduce power consumption of the wearable device 100, so that the remaining battery level of the wearable device 100 can support completion of blood pressure measurement within one day.

In a possible implementation, the wearable device 100 may automatically reduce the total quantity of times of blood pressure measurement in the blood pressure measurement scheme based on the remaining battery level of the wearable device 100, so that the remaining battery level of the wearable device 100 can support completion of a total quantity of times of blood pressure measurement in a modified blood pressure measurement scheme.

In another possible implementation, the wearable device 100 may receive a user operation to reduce the total quantity of times of blood pressure measurement in the blood pressure measurement scheme, so that the remaining battery level of the wearable device 100 can support completion of a total quantity of times of blood pressure measurement in a modified blood pressure measurement scheme.

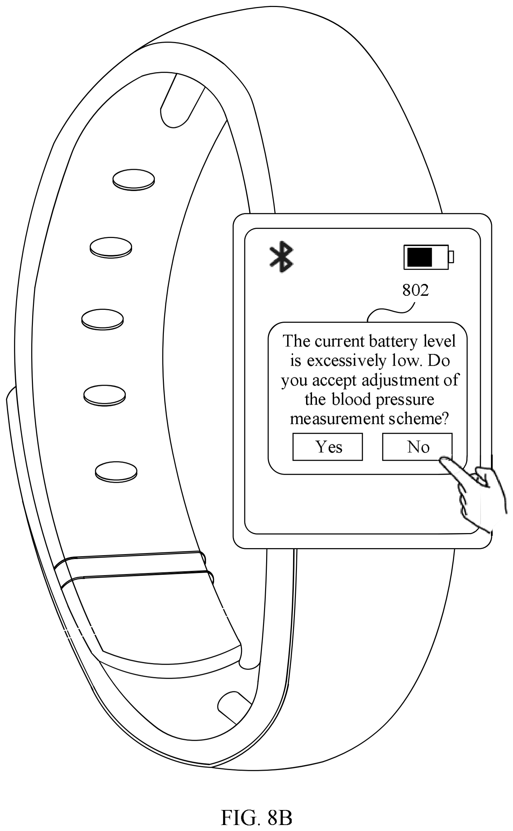

In some embodiments, after the wearable device 100 displays the prompt information 801, the wearable device 100 may determine whether the wearable device 100 is being charged. When detecting, within a first duration (for example, 1 minute), that the wearable device 100 is not being charged, the wearable device 100 may display prompt information 802 shown in FIG. 8B. The prompt information 802 includes text information: "The current battery level is excessively low. Do you accept adjustment of the blood pressure measurement scheme?" The prompt information 802 is used to prompt the user to accept adjustment of the blood pressure measurement scheme, to reduce the quantity of times of blood pressure measurement. The prompt information 802 further includes a Yes option and a No option. The user may determine, by using the Yes option, to accept adjustment of the blood pressure measurement scheme. Alternatively, the user may not accept, by using the No option, adjustment of the blood pressure measurement scheme.

When the user agrees to change the blood pressure measurement scheme, S708 is performed.

When the user does not agree to change the blood pressure measurement scheme, S709 is performed.

S708: The wearable device 100 saves a modified blood pressure measurement scheme.

When the user agrees to change the blood pressure measurement scheme, the wearable device 100 may save the modified blood pressure measurement scheme.

S709: The wearable device 100 prompts the user to enter a low power mode, and determines whether the user agrees to enter the low power mode.

When the user does not agree to change the blood pressure measurement scheme, the wearable device 100 may prompt the user to enter the low power mode, to reduce power consumption of the wearable device 100 and save battery power. The wearable device 100 needs to determine whether the user agrees to enter the low power mode.

Controlling the wearable device 100 to enter the low power mode may be disabling some or all of energy-consuming operations on the wearable device 100 that are irrelevant to blood pressure monitoring. For example, the energy-consuming operations may include but are not limited to a heart rate monitoring function, a motion data recording function, and the like.

When the user allows the wearable device 100 to enter the low power mode, S705 is performed.

When the user does not allow the wearable device 100 to enter the low power mode, S710 is performed.

For example, as shown in FIG. 8B, the wearable device 100 may receive an input operation (for example, tapping) of the user for the No option in the prompt information 802, and in response to the input operation of the user, the wearable device 100 does not adjust the blood pressure measurement scheme.

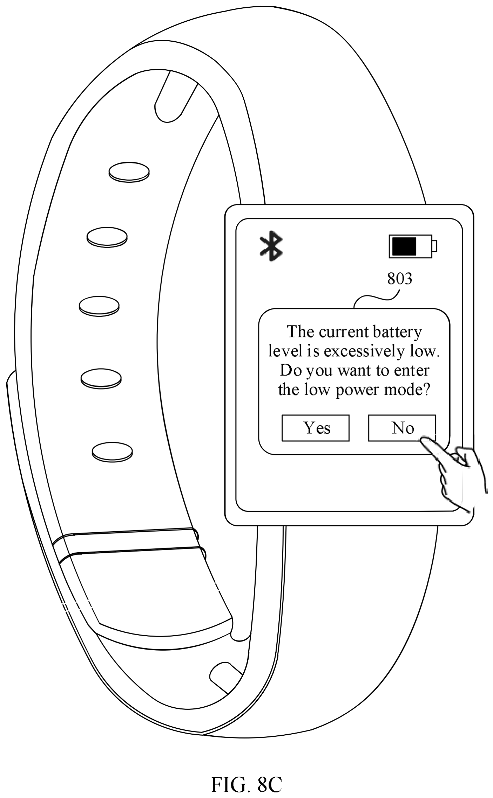

After the user chooses not to adjust the blood pressure measurement scheme, the wearable device 100 may display prompt information 803 shown in FIG. 8C. The prompt information 803 includes the prompt information 803. The prompt information 803 includes text information: "The current battery level is excessively low. Do you want to enter the low power mode?" The prompt information 803 is used to prompt the user to control the wearable device 100 to enter the low power mode, to reduce power consumption of the wearable device 100 and save battery power. The prompt information 803 further includes a Yes option and a No option. The user may control, by using the Yes option, the wearable device 100 to enter the low power mode. Alternatively, the user may control, by using the No option, the wearable device 100 not to enter the low power mode.

S710: The wearable device 100 notifies the user that a battery level is excessively low and the wearable device 100 may be powered off at any time.

When the user does not allow the wearable device 100 to enter the low power mode, the wearable device 100 may notify the user that the battery level is excessively low and the wearable device 100 may be powered off at any time.

For example, as shown in FIG. 8C, the wearable device 100 may receive an input operation (for example, tapping) of the user for the No option in the prompt information 803, and in response to the input operation of the user, the wearable device 100 does not enter the low power mode.

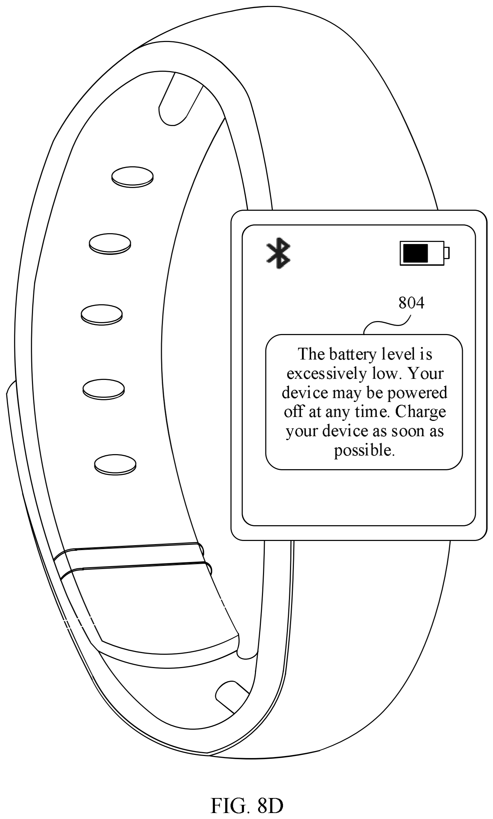

In response to the input operation of the user for the No option in the prompt information 803, the wearable device 100 may display prompt information 804 shown in FIG. 8D. The prompt information 804 may include text information: "The battery level is excessively low. Your device may be powered off at any time. Charge your device as soon as possible." The text information is intended to notify the user that the battery level of the wearable device 100 is excessively low.

Optionally, in the embodiment of FIG. 7A and FIG. 7B, before blood pressure measurement within one day is started, whether the battery level of the wearable device 100 is excessively low may be determined, so that an appropriate policy is selected, to reduce power consumption of the wearable device 100 and increase a use duration of the remaining battery level of the wearable device 100.

**A wearable device 100 uses a blood pressure measurement scheme and displays a result of single blood pressure measurement.**

After obtaining a blood pressure measurement scheme, the wearable device 100 may monitor blood pressure of a user within one day based on the blood pressure measurement scheme.

It can be learned from the foregoing descriptions that the blood pressure measurement scheme may include one or more blood pressure measurement policies. The blood pressure measurement policy may include but is not limited to: a start moment of blood pressure measurement, an end moment of blood pressure measurement, a duration of blood pressure measurement, a quantity of times of blood pressure measurement within the duration of blood pressure measurement, a time interval between two adj acent times of blood pressure measurement within the duration of blood pressure measurement, a total quantity of times of blood pressure measurement, and the like.

In some embodiments, before measuring blood pressure by using the blood pressure measurement scheme, the wearable device 100 needs to determine whether a current user status meets a blood pressure measurement condition. When the blood pressure measurement condition is met, the wearable device 100 starts to measure blood pressure of the user. When the blood pressure measurement condition is not met, the wearable device 100 does not measure blood pressure of the user. The wearable device 100 starts to measure blood pressure of the user only when the blood pressure measurement condition is met.

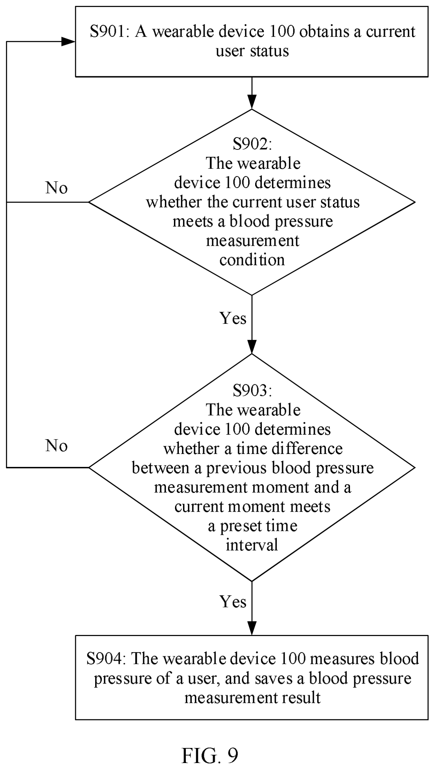

**FIG. 9** **is a schematic flowchart of a method for measuring blood pressure of a user by a wearable device 100.**

S901: The wearable device 100 obtains a current user status.

The current user status may include but is not limited to one or more of the following: motion data, physiological data, and the like.

The motion data may be acceleration data, angular velocity data, a cumulative quantity of steps, and the like.

The physiological data may be a real-time heart rate, a nighttime resting heart rate, and the like. The nighttime resting heart rate may be an average value of heart rates measured by the wearable device 100 at nighttime.

S902: The wearable device 100 determines whether the current user status meets a blood pressure measurement condition.

The blood pressure measurement condition may include but is not limited to one or more of the following: The user is in a stationary state, and a difference between the real-time heart rate and the nighttime resting heart rate is within a preset range.

The wearable device 100 may collect motion data based on a motion sensor. The motion sensor may include but is not limited to an acceleration sensor, a gyroscope sensor, and the like. The motion sensor may collect motion data or a motion trajectory. The wearable device 100 may determine, based on the motion data or the motion trajectory, whether the user is in the stationary state.

Usually, when the user is in the stationary state, the user almost has no activity, and the real-time heart rate is stable. If the difference between the real-time heart rate and the nighttime resting heart rate is within the preset range, it can be determined that the user is in the stationary state. The wearable device 100 may determine, based on the real-time heart rate collected by a PPG sensor, whether the user is in the stationary state.

When it is determined that the current user status meets the blood pressure measurement condition, S903 is performed.

When it is determined that the current user status does not meet the blood pressure measurement condition, S901 is performed.

In this way, before measuring blood pressure, the wearable device 100 may determine whether the blood pressure measurement condition is met. When the blood pressure measurement condition is met, the wearable device 100 starts to measure blood pressure of the user, so that accuracy of blood pressure measurement can be improved. A reason is as follows: When the blood pressure measurement condition is not met, the user may be in a non-stationary state, for example, in a motion state like running or exercising. In this case, a blood pressure result of the wearable device 100 has an error.

S903: The wearable device 100 determines whether a time difference between a previous blood pressure measurement moment and a current moment meets a preset time interval.

When determining that the current user status meets the blood pressure measurement condition, before starting to measure blood pressure, the wearable device 100 may determine whether the time difference between the previous blood pressure measurement moment and the current moment meets the preset time interval.

When it is determined that the time difference between the previous blood pressure measurement moment and the current moment meets the preset time interval, S904 is performed.

When it is determined that the time difference between the previous blood pressure measurement moment and the current moment does not meet the preset time interval, S901 is performed.

It can be learned from the foregoing descriptions that a blood pressure measurement scheme determined by the wearable device 100 may include one or more blood pressure measurement policies. The blood pressure measurement policy may include but is not limited to: a start moment of blood pressure measurement, an end moment of blood pressure measurement, a duration of blood pressure measurement, a quantity of times of blood pressure measurement within the duration of blood pressure measurement, a time interval between two adjacent times of blood pressure measurement within the duration of blood pressure measurement, a total quantity of times of blood pressure measurement, and the like.

Before starting to measure blood pressure, the wearable device 100 needs to determine whether the time difference between the previous blood pressure measurement moment and the current moment is a preset time interval specified in the blood pressure measurement scheme.

The preset time interval varies within different durations of blood pressure measurement. For example, the preset time interval may be 20 minutes at 9:00 a.m. to 12:00 noon, and the preset time interval may be 30 minutes at 9:00 p.m. to 9:00 a.m.

Before starting to measure blood pressure, the wearable device 100 may determine whether the time difference between the previous blood pressure measurement moment and the current moment meets the preset time interval. When the preset time interval is met, the wearable device 100 starts to measure blood pressure of the user, to avoid frequent blood pressure measurement.

S904: The wearable device 100 measures blood pressure of the user, and saves a blood pressure measurement result.

When determining that the time difference between the previous blood pressure measurement moment and the current moment meets the preset time interval, the wearable device 100 may start to measure blood pressure of the user, and save a blood pressure measurement result.

In some embodiments, to ensure accuracy of blood pressure measurement, the wearable device 100 may display prompt information to prompt the user to measure blood pressure.

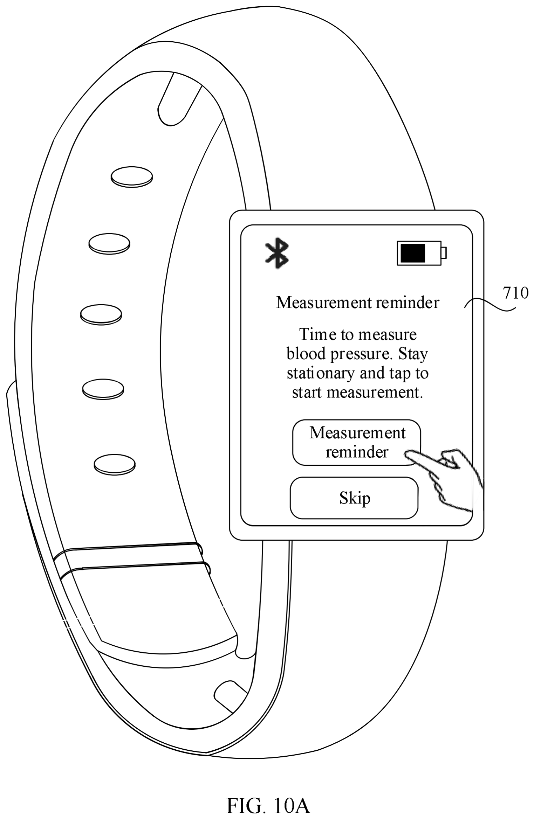

FIG. 10A to FIG. 10E are diagrams of displaying, by a wearable device 100, prompt information to prompt a user to measure blood pressure.

For example, as shown in FIG. 10A, when the wearable device 100 starts to measure blood pressure, to ensure accuracy of a blood pressure measurement result, the wearable device 100 may display a user interface 710 shown in FIG. 10A. The following prompt information is displayed on the user interface 710: "Time to measure blood pressure. Stay stationary and tap to start measurement." The prompt information is used to prompt the user to remain in a stationary state during blood pressure measurement, to avoid inaccuracy of a blood pressure measurement result due to motion. The user interface 710 further includes a Measurement reminder option and a Skip option. The user may view precautions for blood pressure measurement by using the Measurement reminder option. Alternatively, the user may directly start to measure blood pressure without viewing the precautions for blood pressure measurement by using the Skip option.

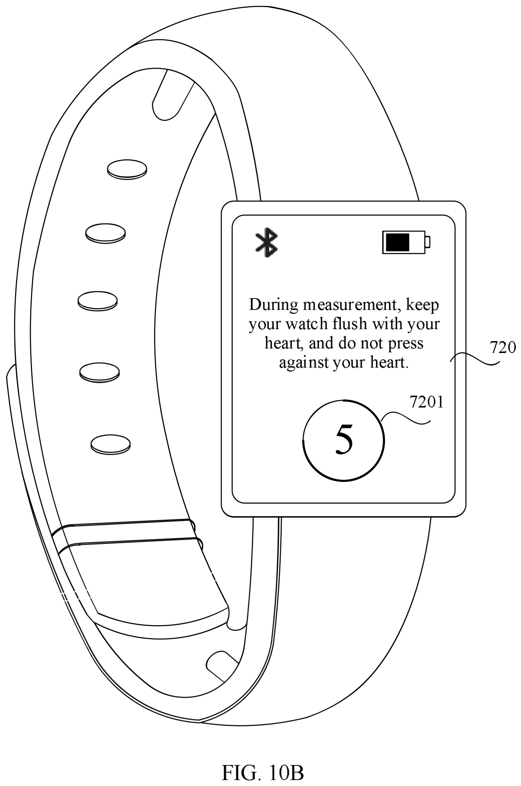

For example, as shown in FIG. 10A, the wearable device 100 may receive an input operation (for example, tapping) of the user for the Measurement reminder option on the user interface 710, and in response to the input operation of the user, the wearable device 100 may display a user interface 720 shown in FIG. 10B. The user interface 720 includes prompt information: "During measurement, keep your watch flush with your heart, and do not press against your heart." The prompt information is used to inform the user of a correct measurement posture. The user interface 720 includes a Timer option 7201. The Timer option 7201 is used to prompt the user to raise the watch to a position flush with the heart within preset time.

After countdown time displayed on the wearable device 100 reaches 0, the wearable device 100 may start to measure blood pressure of the user.

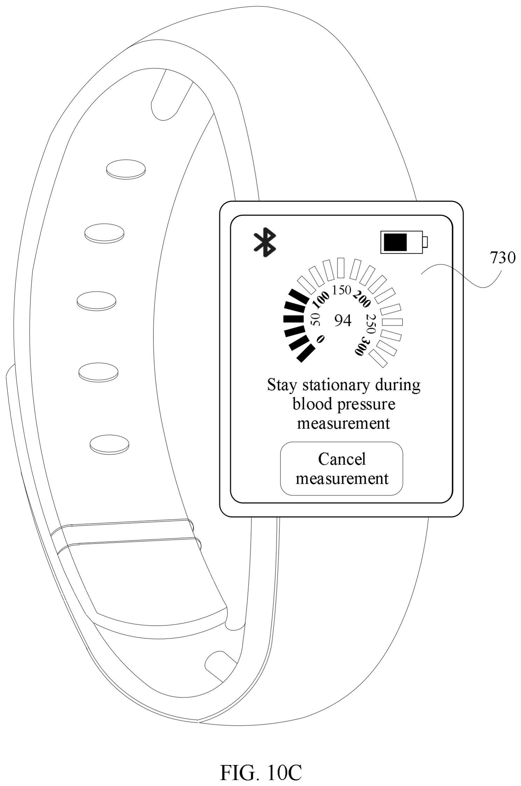

Optionally, in a process in which the wearable device 100 measures blood pressure of the user, the wearable device 100 may display a user interface 730 shown in FIG. 10C. The user interface 730 includes prompt information: "Stay stationary during blood pressure measurement", to prompt the user to remain stationary during blood pressure measurement, to avoid inaccuracy of a blood pressure measurement result due to motion. The user interface 730 further includes a Cancel measurement option. The user may stop the blood pressure measurement by using the Cancel measurement option.

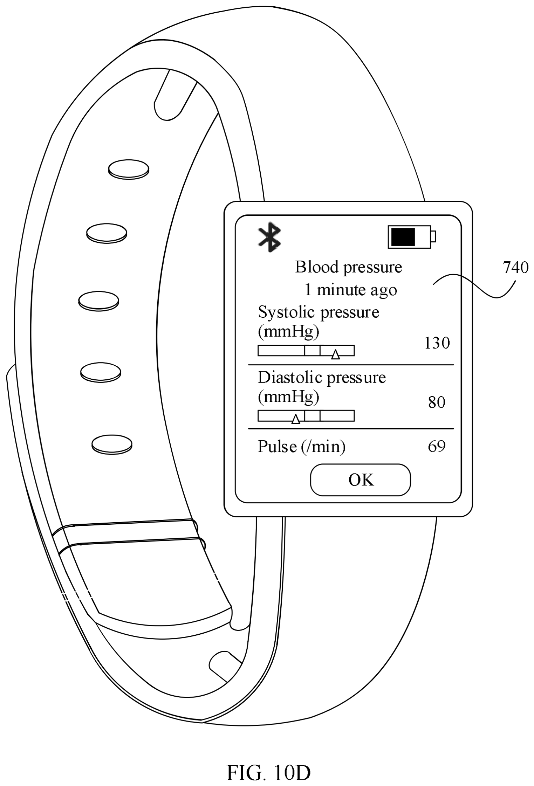

In some embodiments, after the wearable device 100 obtains a blood pressure monitoring value, the wearable device 100 may display a user interface 740 shown in FIG. 10D. The user interface 740 includes the blood pressure measurement value. The blood pressure measurement value may include systolic pressure and diastolic pressure. For example, the systolic pressure may be 130 mmHg, and the diastolic pressure may be 80 mmHg. In some embodiments, the user interface 740 may further include a pulse. For example, the pulse may be 69 times per minute.

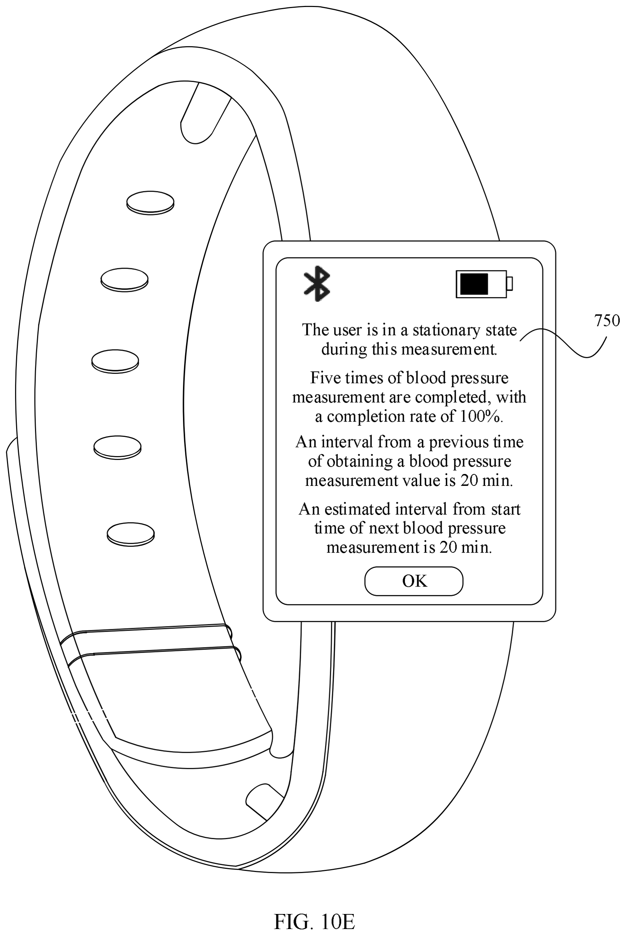

In some embodiments, after the wearable device 100 obtains a blood pressure monitoring value, the wearable device 100 may alternatively display a user interface 750 shown in FIG. 10E. Detailed information of the blood pressure measurement, for example, a user status, a total quantity of times of blood pressure measurement, a time interval from previous blood pressure measurement, and a time interval from next blood pressure measurement, is displayed on the user interface 750. For example, as shown in FIG. 10E, the user status is a stationary state, the total quantity of times of blood pressure measurement is 5 times, an interval from a previous time of obtaining a blood pressure measurement value is 20 minutes, and an estimated interval from start time of next blood pressure measurement is also 20 minutes.

The user interface 750 further includes an OK option and an Optimize blood pressure measurement scheme option. The user may enable, by using the OK option, the wearable device 100 to stop displaying the user interface 750. Alternatively, the user may actively modify a blood pressure measurement scheme by using the Optimize blood pressure measurement scheme option. For details, refer to descriptions in the following embodiments. Details are not described herein in this application.

In some embodiments, the wearable device 100 may alternatively not display the prompt information shown in FIG. 10A to FIG. 10E, but directly start to measure blood pressure of the user, to avoid frequently disturbing the user. For example, at nighttime, the wearable device 100 may not display the prompt information shown in FIG. 10A to FIG. 10E, and the wearable device 100 may automatically measure blood pressure of the user without displaying the prompt information, to avoid disturbing resting of the user.

In some embodiments, a wearable device 100 may measure blood pressure of a user, and obtain a blood pressure measurement result. Before saving the blood pressure measurement result, the wearable device 100 needs to determine whether a current user status meets a blood pressure measurement condition. When the blood pressure measurement condition is met, the wearable device 100 may directly save the blood pressure measurement result. When the blood pressure measurement condition is not met, the wearable device 100 may determine a blood pressure compensation value based on the current user status, correct the blood pressure measurement result based on the blood pressure compensation value, and save a corrected blood pressure measurement result.

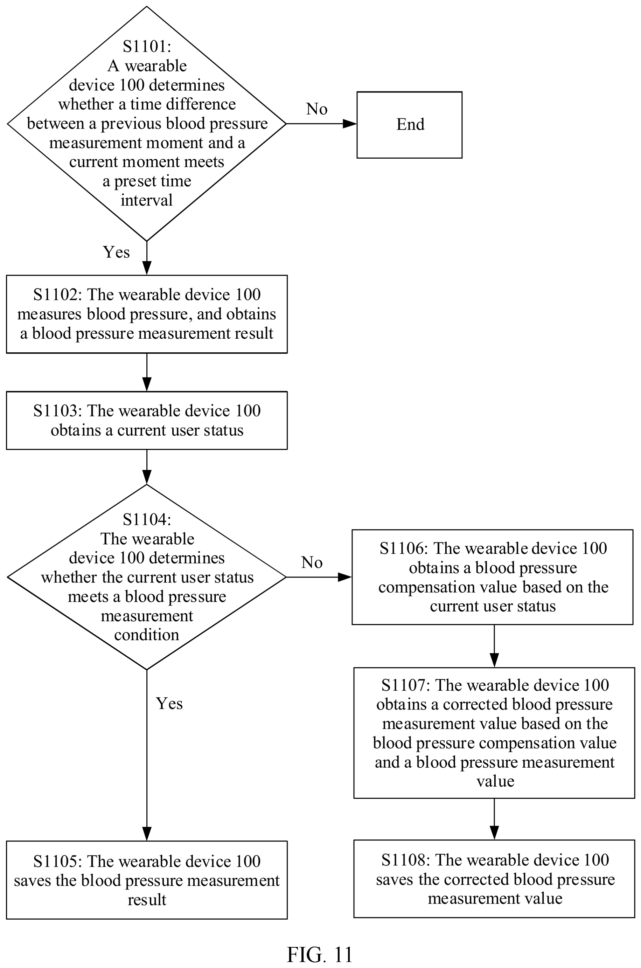

**FIG. 11** **is a schematic flowchart of another method for measuring blood pressure of a user by a wearable device 100.**

S1101: The wearable device 100 determines whether a time difference between a previous blood pressure measurement moment and a current moment meets a preset time interval.

Before starting to measure blood pressure, the wearable device 100 needs to determine whether the time difference between the previous blood pressure measurement moment and the current moment meets the preset time interval.

When it is determined that the time difference between the previous blood pressure measurement moment and the current moment meets the preset time interval, S1102 is performed.

When it is determined that the time difference between the previous blood pressure measurement moment and the current moment does not meet the preset time interval, the process ends, and blood pressure is not measured, until the time difference between the previous blood pressure measurement moment and the current moment meets the preset time interval.

It can be learned from the foregoing descriptions that a blood pressure measurement scheme determined by the wearable device 100 may include one or more blood pressure measurement policies. The blood pressure measurement policy may include but is not limited to: a start moment of blood pressure measurement, an end moment of blood pressure measurement, a duration of blood pressure measurement, a quantity of times of blood pressure measurement within the duration of blood pressure measurement, a time interval between two adjacent times of blood pressure measurement within the duration of blood pressure measurement, a total quantity of times of blood pressure measurement, and the like.

Before starting to measure blood pressure, the wearable device 100 needs to determine whether the time difference between the previous blood pressure measurement moment and the current moment is a preset time interval specified in the blood pressure measurement scheme.

The preset time interval varies within different durations of blood pressure measurement. For example, the preset time interval may be 20 minutes at 9:00 a.m. to 12:00 noon, and the preset time interval may be 30 minutes at 9:00 p.m. to 9:00 a.m.

Before starting to measure blood pressure, the wearable device 100 may determine whether the time difference between the previous blood pressure measurement moment and the current moment meets the preset time interval. When the preset time interval is met, the wearable device 100 starts to measure blood pressure of the user, to avoid frequent blood pressure measurement.

S1102: The wearable device 100 measures blood pressure, and obtains a blood pressure measurement result.

When determining that the time difference between the previous blood pressure measurement moment and the current moment meets the preset time interval, the wearable device 100 may start to measure blood pressure, and obtain a blood pressure measurement result.

S1103: The wearable device 100 obtains a current user status.

S1104: The wearable device 100 determines whether the current user status meets a blood pressure measurement condition.

The wearable device 100 obtains the current user status, and determines whether the current user status meets the blood pressure measurement condition.

For descriptions of S1103 and S1104, refer to the descriptions of S901 and S902. Details are not described herein again in this application.

When it is determined that the current user status meets the blood pressure measurement condition, S1105 is performed.

When it is determined that the current user status does not meet the blood pressure measurement condition, S1106 is performed.

S1105: The wearable device 100 saves the blood pressure measurement result.

When it is determined that the current user status meets the blood pressure measurement condition, the wearable device 100 may directly save the blood pressure measurement result.

S1106: The wearable device 100 obtains a blood pressure compensation value based on the current user status.

When determining that the current user status does not meet the blood pressure measurement condition, the wearable device 100 may obtain the blood pressure compensation value based on the current user status. The determined blood pressure compensation value varies depending on different user statuses.

S1107: The wearable device 100 obtains a corrected blood pressure measurement value based on the blood pressure compensation value and a blood pressure measurement value.

S1108. The wearable device 100 saves the corrected blood pressure measurement value.

After determining whether the current user status meets the blood pressure measurement condition, the wearable device 100 may obtain the blood pressure compensation value based on the current user status, and correct the blood pressure measurement value based on the blood pressure compensation value, to obtain the corrected blood pressure measurement value. This is intended to eliminate impact of a user status that does not meet the blood pressure measurement condition on the blood pressure measurement value.

After obtaining the corrected blood pressure measurement value, the wearable device 100 may save the corrected blood pressure measurement value.

It can be learned from the embodiment of FIG. 7A and FIG. 7B that, after a wearable device 100 determines a blood pressure measurement scheme and before the wearable device 100 uses the blood pressure measurement scheme, the wearable device 100 may determine a minimum battery level needed by the blood pressure measurement scheme. When a remaining battery level of the wearable device 100 is excessively low, a user may charge the wearable device 100 as soon as possible, to avoid a case in which the wearable device 100 is powered off due to an excessively low battery level and cannot measure blood pressure.

In some embodiments, before measuring blood pressure, the wearable device 100 needs to detect whether the wearable device 100 is in a worn state. When the wearable device 100 is in a non-worn state, the user is prompted, in a timely manner, to wear the wearable device 100 and start to measure blood pressure.

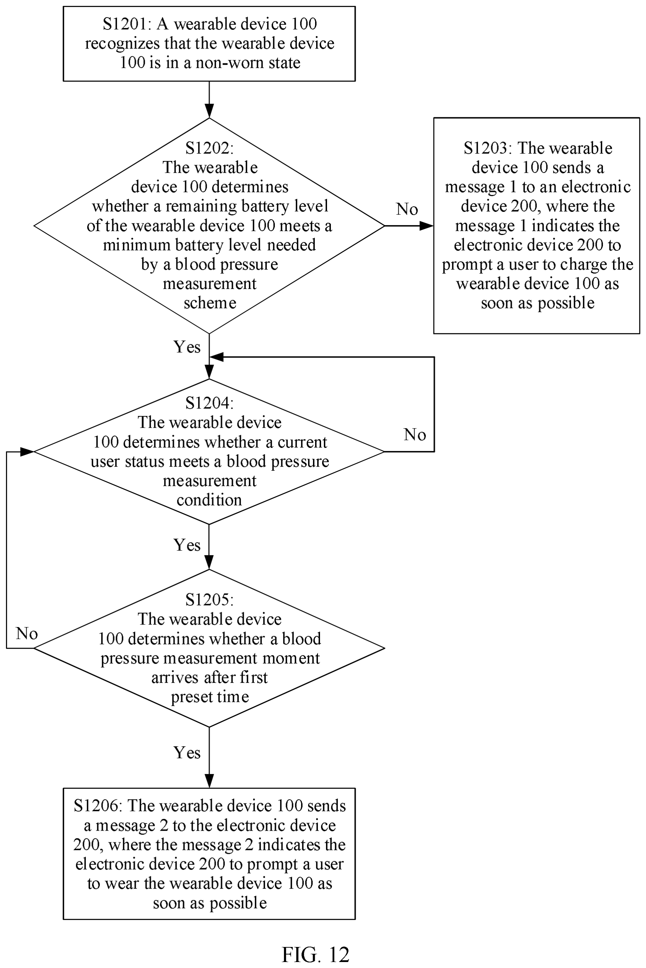

**FIG. 12** **is a schematic flowchart of still another method for measuring blood pressure of a user by a wearable device 100.**

S1201: The wearable device 100 recognizes that the wearable device 100 is in a non-worn state.

S1202: The wearable device 100 determines whether a remaining battery level of the wearable device 100 meets a minimum battery level needed by a blood pressure measurement scheme.

The wearable device 100 may determine whether the wearable device 100 is worn on a wrist of the user. When the wearable device 100 is not worn on a wrist of the user, the wearable device 100 determines that the wearable device 100 is in the non-worn state.

The wearable device 100 may obtain the remaining battery level of the wearable device 100, and determine, based on the blood pressure measurement scheme, the minimum battery level needed for completing the blood pressure measurement scheme.

The wearable device 100 needs to determine whether the remaining battery level of the wearable device 100 is greater than the minimum battery level needed by the blood pressure measurement scheme.

When the remaining battery level of the wearable device 100 is less than the minimum battery level needed by the blood pressure measurement scheme, S1203 is performed.

When the remaining battery level of the wearable device 100 is greater than the minimum battery level needed by the blood pressure measurement scheme, S1204 is performed.

S1203: The wearable device 100 sends a message 1 to an electronic device 200, where the message 1 indicates the electronic device 200 to prompt the user to charge the wearable device 100 as soon as possible.

When the remaining battery level of the wearable device 100 is less than the minimum battery level needed by the blood pressure measurement scheme, to avoid a case in which the wearable device 100 is powered off due to an excessively low battery level and cannot measure blood pressure, the wearable device 100 may send the message 1 to the electronic device 200 that has established a communication connection to the wearable device 100, where the message 1 indicates the electronic device 200 to prompt the user to charge the wearable device 100 as soon as possible.

After the electronic device 200 receives the message 1 sent by the wearable device 100, the electronic device 200 may prompt the user to charge the wearable device 100 as soon as possible.

Optionally, a prompt manner may include but is not limited to: vibration, voice, a pop-up window, blinking light, and the like.

Optionally, the electronic device 200 may use different prompt manners depending on different use statuses.

For example, when the electronic device 200 is in an in-use state, the electronic device 200 may prompt, by using a pop-up window, the user to charge the wearable device 100 as soon as possible.

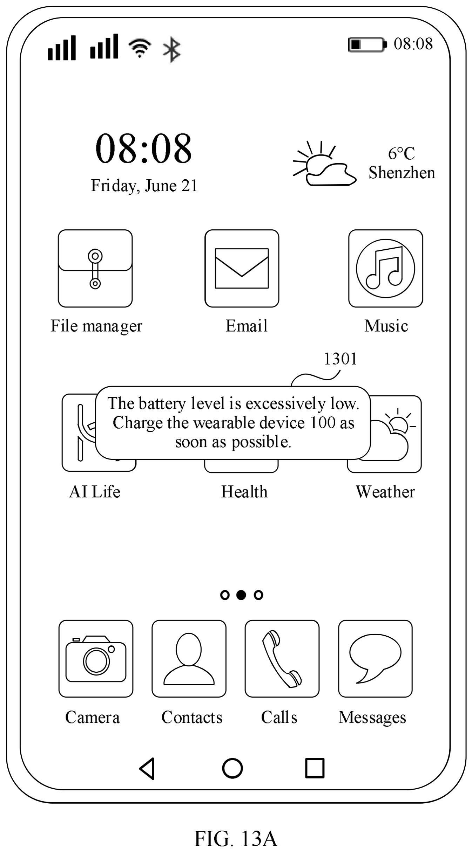

For example, as shown in FIG. 13A, the electronic device 200 may display a prompt bar 1301 above a home screen in a floating manner, and the prompt bar 1301 includes prompt information: "The battery level is excessively low. Charge the wearable device 100 as soon as possible." The prompt bar 1301 is used to prompt the user to charge the wearable device 100 as soon as possible.

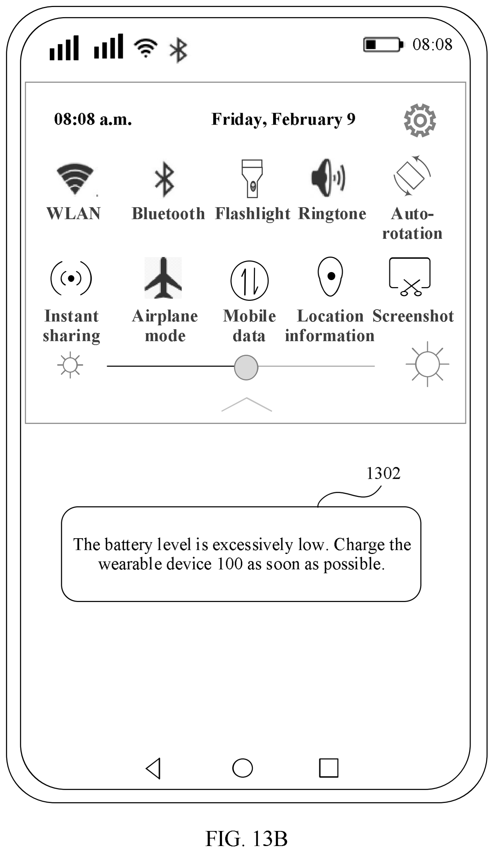

For example, as shown in FIG. 13B, the electronic device 200 may display prompt information 1302 in a drop-down notification bar. The prompt information 1302 includes the following prompt information: "The battery level is excessively low. Charge the wearable device 100 as soon as possible." The prompt information 1302 is used to prompt the user to charge the wearable device 100 as soon as possible.

In addition to the manner shown in FIG. 13A and FIG. 13B in which the electronic device 200 displays the prompt information to prompt the user to charge the wearable device 100 as soon as possible, the electronic device 200 may alternatively prompt, in another manner, the user to charge the wearable device 100 as soon as possible. Details are not described herein in this embodiment of this application.

For another example, when the electronic device 200 is in an unused state, the electronic device 200 may prompt, in one or more manners such as vibration, voice, and blinking light, the user to charge the wearable device 100 as soon as possible.

S1204: The wearable device 100 determines whether a current user status meets a blood pressure measurement condition.

When the remaining battery level of the wearable device 100 is greater than the minimum battery level needed by the blood pressure measurement scheme, the wearable device 100 needs to determine whether the current user status meets the blood pressure measurement condition. When the current user status meets the blood pressure measurement condition, the wearable device 100 starts to measure blood pressure of the user.

For descriptions of S1204, refer to the descriptions of S901 and S902. Details are not described herein again in this application.

When it is determined that the current user status meets the blood pressure measurement condition, S1205 is performed.

When it is determined that the current user status does not meet the blood pressure measurement condition, a user status is continuously monitored, until the user status meets the blood pressure measurement condition.

S1205: The wearable device 100 determines whether a blood pressure measurement moment arrives after first preset time.

When determining that the current user status meets the blood pressure measurement condition, before measuring blood pressure, the wearable device 100 may determine whether the blood pressure measurement moment arrives after the first preset time. In this way, the electronic device 200 can prompt, in advance and in a timely manner, the user to wear the wearable device 100 to measure blood pressure.

When it is determined that the blood pressure measurement moment arrives after the first preset time, S1206 is performed, so that the electronic device 200 can prompt, in advance and in a timely manner, the user to wear the wearable device 100 to measure blood pressure.

When it is determined that the blood pressure measurement moment does not arrive after the first preset time, S1204 is performed.

S1206: The wearable device 100 sends a message 2 to the electronic device 200, where the message 2 indicates the electronic device 200 to prompt the user to wear the wearable device 100 as soon as possible.

When the wearable device 100 determines that the blood pressure measurement moment does not arrive after the first preset time, to enable the electronic device 200 to prompt, in advance and in a timely manner, the user to wear the wearable device 100, the wearable device 100 may send the message 2 to the electronic device 200 that has established a communication connection to the wearable device 100, where the message 2 indicates the electronic device 200 to prompt the user to wear the wearable device 100 as soon as possible.

After receiving the message 2 sent by the wearable device 100, the electronic device 200 may prompt the user to wear the wearable device 100 as soon as possible.

Optionally, a prompt manner may include but is not limited to: vibration, voice, a pop-up window, blinking light, and the like.

Optionally, the electronic device 200 may use different prompt manners depending on different use statuses.

For example, when the electronic device 200 is in an in-use state, the electronic device 200 may prompt, by using a pop-up window, the user to wear the wearable device 100 as soon as possible.

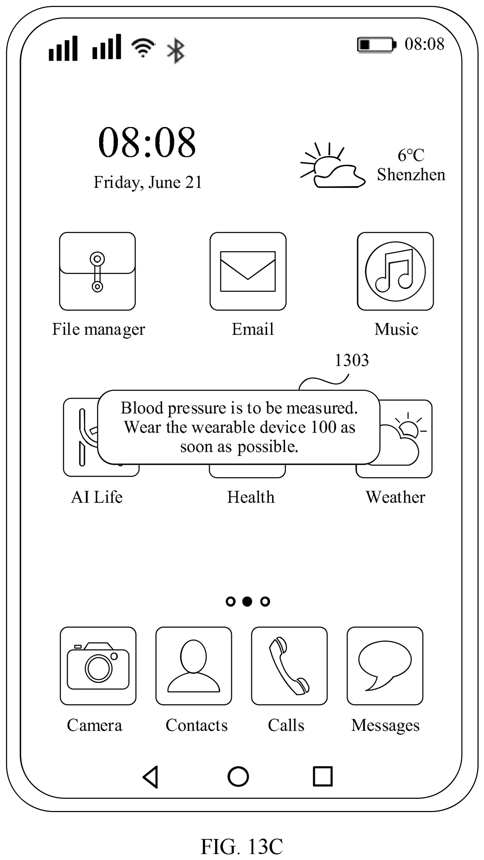

For example, as shown in FIG. 13C, the electronic device 200 may display a prompt bar 1303 above a home screen in a floating manner, and the prompt bar 1303 includes prompt information: "Blood pressure is to be measured. Wear the wearable device 100 as soon as possible." The prompt bar 1301 is used to prompt the user to wear the wearable device 100 as soon as possible.

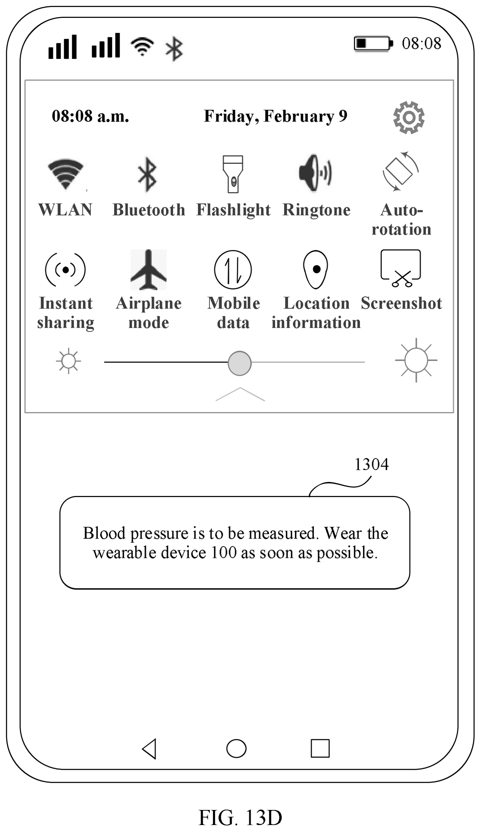

For example, as shown in FIG. 13D, the electronic device 200 may display prompt information 1304 in a drop-down notification bar. The prompt information 1304 includes the following prompt information: "Blood pressure is to be measured. Wear the wearable device 100 as soon as possible." The prompt information 1304 is used to prompt the user to wear the wearable device 100 as soon as possible.

In addition to the manner shown in FIG. 13C and FIG. 13D in which the electronic device 200 displays the prompt information to prompt the user to wear the wearable device 100 as soon as possible, the electronic device 200 may alternatively prompt, in another manner, the user to wear the wearable device 100 as soon as possible. Details are not described herein in this embodiment of this application.

For another example, when the electronic device 200 is in an unused state, the electronic device 200 may prompt, in one or more manners such as vibration, voice, and blinking light, the user to wear the wearable device 100 as soon as possible.

**After completing blood pressure measurement within one cycle by using a blood pressure measurement scheme, a wearable device 100 displays a blood pressure measurement result within the cycle.**

For example, 24 hours may be one cycle.

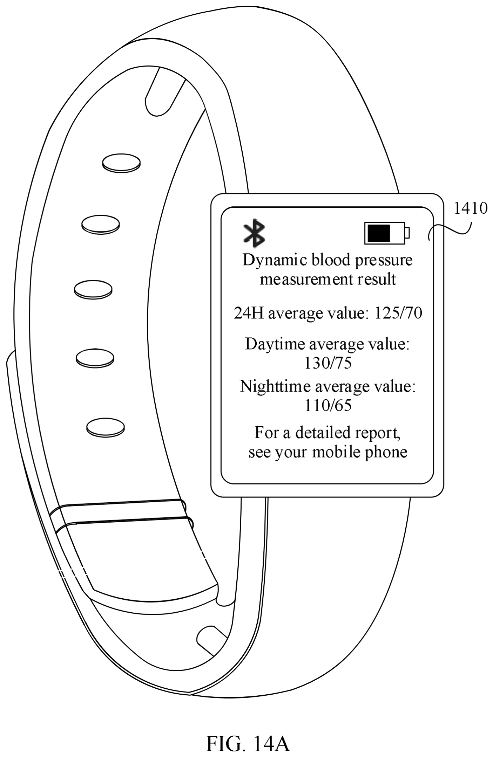

**FIG. 14A to FIG. 14D** **are diagrams of displaying, by a wearable device 100 or an electronic device 200, a blood pressure measurement result within one cycle.**

For example, after the wearable device 100 completes blood pressure measurement within one cycle, the wearable device 100 may display a user interface 1410 shown in FIG. 14A. The user interface 1410 includes a blood pressure measurement result within one cycle. For example, average systolic pressure within 24 hours is 125 mmHg, average diastolic pressure within 24 hours is 70 mmHg, average systolic pressure at daytime is 130 mmHg, average diastolic pressure at daytime is 75 mmHg, average systolic pressure at nighttime is 110 mmHg, and average diastolic pressure at nighttime is 65 mmHg. The following prompt information is further displayed on the user interface 1410: "For a detailed report, see your mobile phone". The user may view, on the electronic device 200, detailed information of a blood pressure measurement result within one cycle.

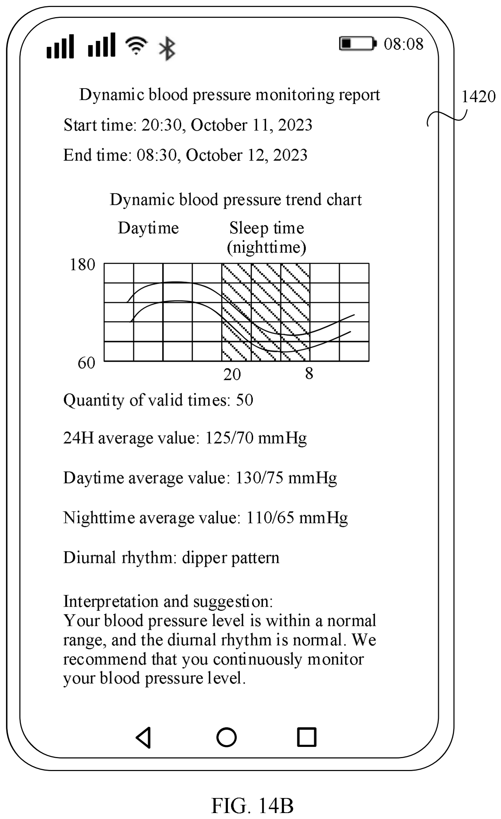

For example, FIG. 14B shows a user interface 1420 on the electronic device 200 on which details of blood pressure measurement within one cycle are displayed. As shown in FIG. 14B, the user interface 1420 includes time to fall asleep, time to get up, and a dynamic blood pressure trend chart within one cycle. The time to fall asleep may be 8:30 p.m. on October 11, 2023. The time to get up may be 8:30 a.m. on October 12, 2023. The dynamic blood pressure trend chart shows a blood pressure change trend within one cycle. The user interface 1420 further includes the following information: A quantity of times of blood pressure measurement is 50 times. Within one cycle (for example, 24 hours), average systolic pressure is 125 mmHg, and average diastolic pressure is 70 mmHg. Within one cycle (for example, 24 hours), average systolic pressure at daytime is 130 mmHg, average diastolic pressure at daytime is 75 mmHg. Within one cycle (for example, 24 hours), average systolic pressure at nighttime is 110 mmHg, and average diastolic pressure at nighttime is 65 mmHg. Diurnal blood pressure rhythm within one cycle (for example, 24 hours) is a dipper pattern. Based on the blood pressure measurement result within one cycle, an interpretation and a suggestion are further displayed on the user interface 1400: Your blood pressure level is within a normal range, and the diurnal rhythm is normal. We recommend that you continuously monitor your blood pressure level.

In another embodiment, when the wearable device 100 does not complete blood pressure measurement within one cycle, for example, completes only a plurality of times of blood pressure measurement within one time period, the user may alternatively view a blood pressure detection result within the time period on the wearable device 100 or the electronic device 200.

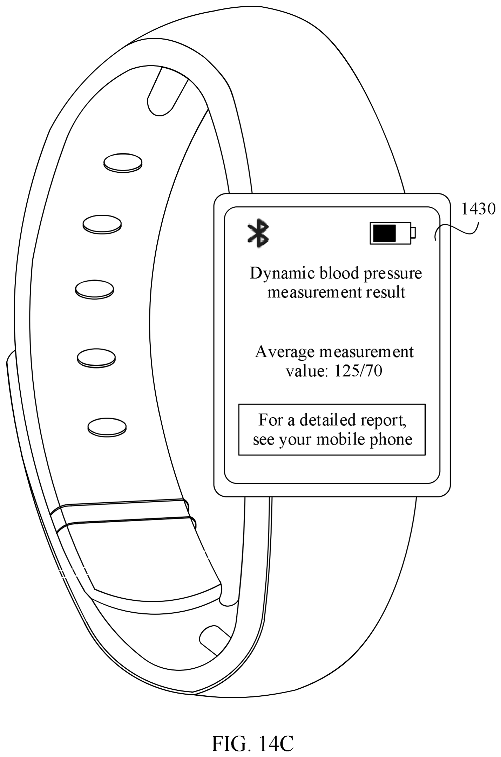

For example, as shown in FIG. 14C, the wearable device 100 displays a user interface 1430, and the user interface 1430 displays an average blood pressure measurement value within the time period. For example, within the time period, average systolic pressure is 125 mmHg, and average diastolic pressure is 70 mmHg. The following prompt information is further displayed on the user interface 1430: "For a detailed report, see your mobile phone". The user may view, on the electronic device 200, detailed information of the blood pressure measurement result within the time period.

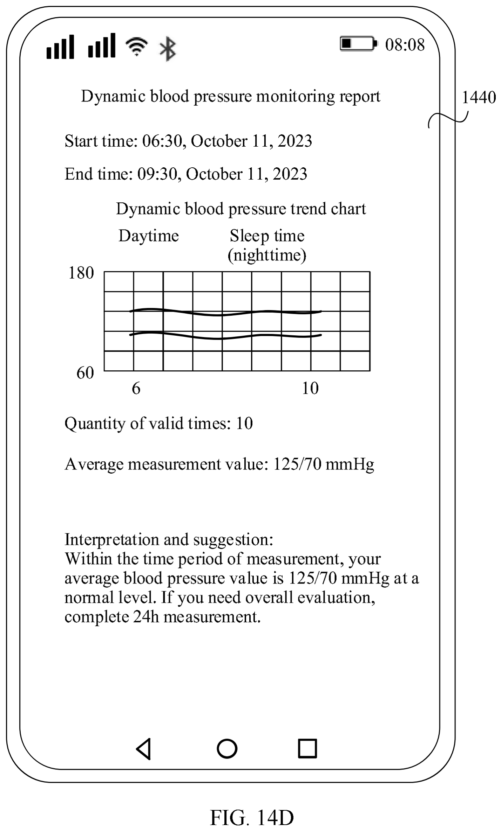

For example, FIG. 14D shows a user interface 1440 on the electronic device 200 on which details of blood pressure measurement within one time period are displayed. As shown in FIG. 14D, the user interface 1440 includes start time of blood pressure measurement, end time of blood pressure measurement, and a dynamic blood pressure trend chart within the time period. The start time of blood pressure measurement may be 6:30 a.m. on October 11, 2023. The end time of blood pressure measurement may be 9:30 a.m. on October 11, 2023. The dynamic blood pressure trend chart shows a blood pressure change trend within one time period. The user interface 1440 further includes the following information: A quantity of times of blood pressure measurement is 10 times. Within the time period, average systolic pressure is 125 mmHg, and average diastolic pressure is 70 mmHg. Based on the blood pressure measurement result within the time period, an interpretation and a suggestion are further displayed on the user interface 1440: Within the time period of measurement, your average blood pressure values are 125/70 mmHg at a normal level. If you need overall evaluation, complete 24-hour measurement.

**A wearable device 100 corrects a blood pressure measurement scheme based on historical blood pressure measurement data, to obtain an optimized blood pressure measurement scheme.**

In some embodiments, the wearable device 100 may obtain historical blood pressure measurement data within a first duration (for example, one month), and correct, based on the historical blood pressure measurement data within the first duration, a blood pressure measurement scheme obtained by the wearable device 100.

The historical blood pressure measurement data includes but is not limited to a historical blood pressure measurement record and a historical blood pressure measurement result. The historical blood pressure measurement record may include but is not limited to: a start moment of historical blood pressure measurement, an end moment of historical blood pressure measurement, a duration of historical blood pressure measurement, a quantity of times of blood pressure measurement within the duration of historical blood pressure measurement, a time interval between two adjacent times of blood pressure measurement within the duration of historical blood pressure measurement, a total quantity of times of historical blood pressure measurement, and the like.

In a possible implementation, the wearable device 100 may periodically or aperiodically correct the blood pressure measurement scheme based on the historical blood pressure measurement data, to obtain an optimized blood pressure measurement scheme. In this way, the optimized blood pressure measurement scheme better conforms to behavior characteristics of a user.

In another possible implementation, the wearable device 100 may alternatively receive a user operation, and correct the blood pressure measurement scheme based on the historical blood pressure measurement data, to obtain an optimized blood pressure measurement scheme. In this way, the optimized blood pressure measurement scheme better conforms to behavior characteristics of a user.

For example, when the wearable device 100 determines, based on the historical blood pressure measurement data, that the user is a user with hypertension, a total quantity of times of blood pressure measurement in the optimized blood pressure measurement scheme determined by the wearable device 100 may be increased, to monitor blood pressure of the user in more detail.

For another example, when the wearable device 100 determines, based on the historical blood pressure measurement data, that the user is a user with normal blood pressure, a total quantity of times of blood pressure measurement in the optimized blood pressure measurement scheme determined by the wearable device 100 may be reduced, to reduce power consumption of the wearable device 100.

For another example, when the wearable device 100 determines, based on the historical blood pressure measurement data, that blood pressure of the user is high at 9:00 a.m. to 11:00 a.m., a total quantity of times of blood pressure measurement at 9:00 a.m. to 11:00 a.m. in the optimized blood pressure measurement scheme determined by the wearable device 100 may be increased, to monitor blood pressure of the user at 9:00 a.m. to 11:00 a.m. in more detail.

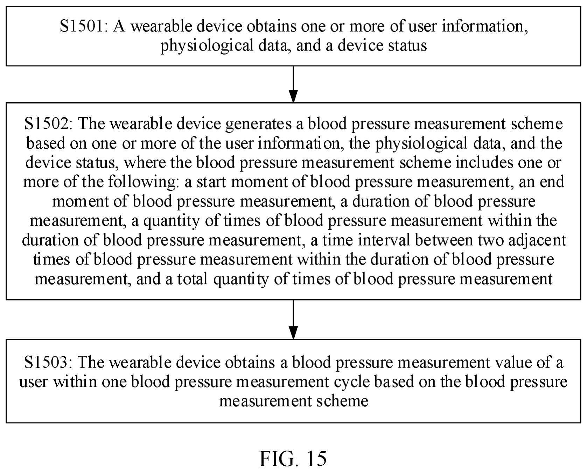

FIG. 15 is a schematic method flowchart of a method for generating a blood pressure measurement scheme according to this application.

S1501: A wearable device obtains one or more of user information, physiological data, and a device status.

S1502: The wearable device generates a blood pressure measurement scheme based on one or more of the user information, the physiological data, and the device status, where the blood pressure measurement scheme includes one or more of the following: a start moment of blood pressure measurement, an end moment of blood pressure measurement, a duration of blood pressure measurement, a quantity of times of blood pressure measurement within the duration of blood pressure measurement, a time interval between two adjacent times of blood pressure measurement within the duration of blood pressure measurement, and a total quantity of times of blood pressure measurement.

S1503: The wearable device obtains a blood pressure measurement value of a user within one blood pressure measurement cycle based on the blood pressure measurement scheme.

For example, one blood pressure measurement cycle may be 24 hours.

According to the method, the wearable device may automatically generate the blood pressure measurement scheme based on fusion of a plurality of types of information, so that the blood pressure measurement scheme generated by the wearable device better conforms to user characteristics. This not only simplifies a user operation, but also improves appropriateness of blood pressure measurement.

In a possible implementation, the user information includes one or more of the following: a gender, an age, a height, a weight, smoking history, alcohol consumption, medical history, medication history, and lifestyle habits; the physiological data includes one or more of the following: a heart rate, blood pressure, arteriosclerosis, and blood glucose; and the device status includes one or more of the following: a remaining battery level and device temperature.

In a possible implementation, that the wearable device generates the blood pressure measurement scheme based on one or more of the user information, the physiological data, and the device status specifically includes: The wearable device determines a risk level of a hypertension risk of the user based on one or more of the user information, the physiological data, and the device status. The wearable device generates the blood pressure measurement scheme based on the risk level of the hypertension risk of the user.

Different blood pressure measurement schemes are generated for different risk levels of hypertension risks. For example, a total quantity of times of blood pressure measurement that is determined by the wearable device based on a high risk is greater than a total quantity of times of blood pressure measurement that is determined by the wearable device based on a low risk.

In some embodiments, the risk level of the hypertension risk may be a risk level within a specific time period within one blood pressure measurement cycle. For example, a risk level of a hypertension risk of the user within a nighttime period is higher than a risk level of a hypertension risk of the user within a daytime period. In this case, a total quantity of times of blood pressure measurement within the nighttime period is greater than a total quantity of times of blood pressure measurement within the daytime period.

In this way, the wearable device may determine the risk level of the hypertension risk of the user based on a plurality of types of information, and then generate the blood pressure measurement scheme based on the risk level of the hypertension risk.

In a possible implementation, after the wearable device generates the blood pressure measurement scheme, the method further includes: The wearable device displays first prompt information, where the first prompt information is used to prompt the user to view detailed content of the blood pressure measurement scheme. The wearable device receives and responds to a first operation of the user for a first option in the first prompt information, and displays a first user interface, where the detailed content of the blood pressure measurement scheme is displayed on the first user interface.

In this way, after the wearable device automatically generates the blood pressure measurement scheme, the wearable device may prompt the user to view the detailed content of the blood pressure measurement scheme. In some embodiments, the wearable device may alternatively receive a user operation to modify the blood pressure measurement scheme.

In a possible implementation, before the wearable device obtains the blood pressure measurement value of the user within the blood pressure measurement cycle through measurement based on the blood pressure measurement scheme, the method further includes: The wearable device obtains the remaining battery level of the wearable device. The wearable device displays second prompt information when the remaining battery level of the wearable device is less than a preset battery level, where the second prompt information is used to prompt the user to charge the wearable device, and the preset battery level is a minimum battery level needed by the wearable device to complete blood pressure measurement within one blood pressure measurement cycle based on the blood pressure measurement scheme. The wearable device saves the blood pressure measurement scheme if the wearable device is in a charging state within a first duration after the second prompt information is displayed.

In this way, before the wearable device measures blood pressure based on the automatically generated blood pressure measurement scheme, the device needs to determine whether the remaining battery level of the wearable device is sufficient, to avoid a case in which the wearable device is powered off before blood pressure measurement within one cycle is completed. When the wearable device displays the prompt information and the wearable device is in the charging state, the wearable device may save the blood pressure measurement scheme, and complete blood pressure measurement within one blood pressure measurement cycle based on the blood pressure measurement scheme.

In a possible implementation, the method further includes: The wearable device displays third prompt information if the wearable device is not in the charging state within the first duration after the second prompt information is displayed, where the third prompt information is used to prompt the user to change the blood pressure measurement scheme. The wearable device receives and responds to a second operation of the user for a second option in the third prompt information, and obtains and saves a first updated blood pressure measurement scheme, where a total quantity of times of blood pressure measurement in the first updated blood pressure measurement scheme is less than the total quantity of times of blood pressure measurement in the blood pressure measurement scheme. The wearable device obtains a blood pressure measurement value of the user within one blood pressure measurement cycle based on the first updated blood pressure measurement scheme.

Optionally, a battery level needed for completing blood pressure measurement within one blood pressure measurement cycle based on the first updated blood pressure measurement scheme is less than or equal to the remaining battery level of the wearable device.

In this way, if the wearable device is still not in the charging state after the wearable device displays the prompt information, the wearable device may re-prompt the user to modify the blood pressure measurement scheme, and obtain the first updated blood pressure measurement scheme.

In a possible implementation, the method further includes: The wearable device receives and responds to a third operation of the user for a third option in the third prompt information, and displays fourth prompt information, where the fourth prompt information is used to prompt the user to set the wearable device to a low power mode. The wearable device receives and responds to a fourth operation of the user for a fourth option in the fourth prompt information, and enters the low power mode.

Optionally, in the low power mode, some or all of energy-consuming operations on the wearable device 100 that are irrelevant to blood pressure monitoring may be disabled. For example, the energy-consuming operations may include but are not limited to a heart rate monitoring function, a motion data recording function, and the like.

In this way, if the user still does not change the blood pressure measurement scheme after the wearable device displays the prompt information, the wearable device may re-prompt the user to control the wearable device to enter the low power mode, to save battery power of the device.

In a possible implementation, the method further includes: The wearable device receives and responds to a fifth operation of the user for a fifth option in the fourth prompt information, and displays fifth prompt information, where the fifth prompt information is used to notify the user that a battery level of the wearable device is excessively low.

In this way, if the wearable device still does not enter the low power mode after the wearable device displays the prompt information, the wearable device may notify the user that the battery level of the wearable device is excessively low and the wearable device may be powered off at any time.

In a possible implementation, the blood pressure measurement value includes a first blood pressure measurement value obtained by the wearable device at a first moment. After the wearable device obtains the first blood pressure measurement value, the method further includes: The wearable device obtains a current user status. When the current user status meets a blood pressure measurement condition, the wearable device saves the first blood pressure measurement value.

In a possible implementation, the method further includes: When the current user status does not meet the blood pressure measurement condition, the wearable device obtains a blood pressure compensation value based on the current user status. The wearable device obtains a first blood pressure monitoring value based on the blood pressure compensation value and the first blood pressure measurement value. The wearable device saves the first blood pressure monitoring value.

The blood pressure measurement condition may include but is not limited to one or more of the following: The user is in a stationary state, and a difference between a real-time heart rate and a nighttime resting heart rate is within a preset range.

The wearable device may collect motion data based on a motion sensor. The motion sensor may include but is not limited to an acceleration sensor, a gyroscope sensor, and the like. The motion sensor may collect motion data or a motion trajectory. The wearable device may determine, based on the motion data or the motion trajectory, whether the user is in the stationary state.

In this way, when measuring blood pressure, the wearable device may determine whether a user status meets the blood pressure measurement condition. When the blood pressure measurement condition is met, the wearable device may directly save a blood pressure measurement result. When the blood pressure measurement condition is not met, the wearable device needs to correct a blood pressure measurement result, and then save a corrected blood pressure measurement result, to improve accuracy of blood pressure measurement.

In a possible implementation, the method further includes: The wearable device obtains historical blood pressure measurement data, where the historical blood pressure measurement data includes a historical blood pressure measurement record and a historical blood pressure measurement result. The wearable device updates the blood pressure measurement scheme based on the historical blood pressure measurement data, and generates and saves a second updated blood pressure measurement scheme. The wearable device obtains a blood pressure measurement value of the user within one blood pressure measurement cycle based on the second updated blood pressure measurement scheme.

The historical blood pressure measurement record includes but is not limited to the historical blood pressure measurement record and the historical blood pressure measurement result. The historical blood pressure measurement record may include but is not limited to: a start moment of historical blood pressure measurement, an end moment of historical blood pressure measurement, a duration of historical blood pressure measurement, a quantity of times of blood pressure measurement within the duration of historical blood pressure measurement, a time interval between two adjacent times of blood pressure measurement within the duration of historical blood pressure measurement, a total quantity of times of historical blood pressure measurement, and the like.

In this way, the wearable device may periodically or aperiodically update the blood pressure measurement scheme based on the historical data of the user, to improve accuracy of the blood pressure measurement scheme generated by the wearable device.

This application provides a wearable device. The wearable device includes a memory and a processor. The memory is coupled to the processor. The memory is configured to store a computer program. When the processor invokes the computer program, the wearable device is enabled to perform the method for generating a blood pressure measurement scheme shown in FIG. 15.

This application provides a computer-readable storage medium, including instructions. When the instructions are run on a wearable device, the wearable device is enabled to perform the method for generating a blood pressure measurement scheme shown in FIG. 15.

This application provides a chip system. The chip system includes one or more processors. The processor is configured to invoke computer instructions, to perform the method for generating a blood pressure measurement scheme shown in FIG. 15.

This application provides a computer program product including instructions. When the computer program product is run on a wearable device, the wearable device is enabled to perform the method for generating a blood pressure measurement scheme shown in FIG. 15.

The foregoing descriptions are merely some embodiments and implementations of this application, but are not intended to limit the protection scope of this application. Any variation or replacement readily figured out by persons skilled in the art within the technical scope disclosed in this application shall fall within the protection scope of this application. Therefore, the protection scope of this application shall be subject to the protection scope of the claims.

It can be understood that the user interfaces described in embodiments of this application are merely example interfaces, and constitute no limitation on the solutions of this application. In other embodiments, the user interfaces may use different interface layouts or may include more or fewer controls, or other function options may be added or omitted. Provided that the user interfaces are based on a same inventive idea provided in this application, all of the user interfaces fall within the protection scope of this application.

It should be noted that, if no contradiction or conflict occurs, any features or any parts of any feature in any embodiment of this application may be combined, and a combined technical solution also falls within the scope of embodiments of this application.

In conclusion, the foregoing embodiments are merely intended to describe the technical solutions of this application, but not to limit this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the scope of the technical solutions in embodiments of this application.

## Claims

1. A method for generating a blood pressure measurement scheme, wherein the method comprises:
obtaining, by a wearable device, one or more of user information, physiological data, and a device status;
generating, by the wearable device, a blood pressure measurement scheme based on one or more of the user information, the physiological data, and the device status, wherein the blood pressure measurement scheme comprises one or more of the following: a start moment of blood pressure measurement, an end moment of blood pressure measurement, a duration of blood pressure measurement, a quantity of times of blood pressure measurement within the duration of blood pressure measurement, a time interval between two adjacent times of blood pressure measurement within the duration of blood pressure measurement, and a total quantity of times of blood pressure measurement; and
obtaining, by the wearable device, a blood pressure measurement value of a user within one blood pressure measurement cycle based on the blood pressure measurement scheme.

2. The method according to claim 1, wherein the user information comprises one or more of the following: a gender, an age, a height, a weight, smoking history, alcohol consumption, medical history, medication history, and lifestyle habits;
the physiological data comprises one or more of the following: a heart rate, blood pressure, arteriosclerosis, and blood glucose; and
the device status comprises one or more of the following: a remaining battery level and device temperature.

3. The method according to claim 1 or 2, wherein generating, by the wearable device, the blood pressure measurement scheme based on one or more of the user information, the physiological data, and the device status specifically comprises:
determining, by the wearable device, a risk level of a hypertension risk of the user based on one or more of the user information, the physiological data, and the device status; and
generating, by the wearable device, the blood pressure measurement scheme based on the risk level of the hypertension risk of the user.

4. The method according to any one of claims 1 to 3, wherein after the wearable device generates the blood pressure measurement scheme, the method further comprises:
displaying, by the wearable device, first prompt information, wherein the first prompt information is used to prompt the user to view detailed content of the blood pressure measurement scheme; and
receiving and responding to, by the wearable device, a first operation of the user for a first option in the first prompt information, and displaying a first user interface, wherein the detailed content of the blood pressure measurement scheme is displayed on the first user interface.

5. The method according to any one of claims 1 to 4, wherein before the wearable device obtains the blood pressure measurement value of the user within the blood pressure measurement cycle through measurement based on the blood pressure measurement scheme, the method further comprises:
obtaining, by the wearable device, the remaining battery level of the wearable device;
displaying, by the wearable device, second prompt information when the remaining battery level of the wearable device is less than a preset battery level, wherein the second prompt information is used to prompt the user to charge the wearable device, and the preset battery level is a minimum battery level needed by the wearable device to complete blood pressure measurement within one blood pressure measurement cycle based on the blood pressure measurement scheme; and
saving the blood pressure measurement scheme if the wearable device is in a charging state within a first duration after the second prompt information is displayed.

6. The method according to claim 5, wherein the method further comprises:
displaying, by the wearable device, third prompt information if the wearable device is not in the charging state within the first duration after the second prompt information is displayed, wherein the third prompt information is used to prompt the user to change the blood pressure measurement scheme;
receiving and responding to, by the wearable device, a second operation of the user for a second option in the third prompt information, and obtaining and saving a first updated blood pressure measurement scheme, wherein a total quantity of times of blood pressure measurement in the first updated blood pressure measurement scheme is less than the total quantity of times of blood pressure measurement in the blood pressure measurement scheme; and
obtaining, by the wearable device, a blood pressure measurement value of the user within one blood pressure measurement cycle based on the first updated blood pressure measurement scheme.

7. The method according to claim 6, wherein the method further comprises:
receiving and responding to, by the wearable device, a third operation of the user for a third option in the third prompt information, and displaying fourth prompt information, wherein the fourth prompt information is used to prompt the user to set the wearable device to a low power mode; and
receiving and responding to, by the wearable device, a fourth operation of the user for a fourth option in the fourth prompt information, and entering the low power mode.

8. The method according to claim 7, wherein the method further comprises:
receiving and responding to, by the wearable device, a fifth operation of the user for a fifth option in the fourth prompt information, and displaying fifth prompt information, wherein the fifth prompt information is used to notify the user that a battery level of the wearable device is excessively low.

9. The method according to any one of claims 1 to 8, wherein the blood pressure measurement value comprises a first blood pressure measurement value obtained by the wearable device at a first moment, and after the wearable device obtains the first blood pressure measurement value, the method further comprises:
obtaining, by the wearable device, a current user status; and
when the current user status meets a blood pressure measurement condition, saving, by the wearable device, the first blood pressure measurement value.

10. The method according to claim 9, wherein the method further comprises:
when the current user status does not meet the blood pressure measurement condition, obtaining, by the wearable device, a blood pressure compensation value based on the current user status;
obtaining, by the wearable device, a first blood pressure monitoring value based on the blood pressure compensation value and the first blood pressure measurement value; and
saving, by the wearable device, the first blood pressure monitoring value.

11. The method according to any one of claims 1 to 10, wherein the method further comprises:
obtaining, by the wearable device, historical blood pressure measurement data, wherein the historical blood pressure measurement data comprises a historical blood pressure measurement record and a historical blood pressure measurement result;
updating, by the wearable device, the blood pressure measurement scheme based on the historical blood pressure measurement data, and generating and saving a second updated blood pressure measurement scheme; and
obtaining, by the wearable device, a blood pressure measurement value of the user within one blood pressure measurement cycle based on the second updated blood pressure measurement scheme.

12. A wearable device, wherein the wearable device comprises a memory and a processor, the memory is coupled to the processor, the memory is configured to store a computer program, and when the processor invokes the computer program, the wearable device is enabled to perform the method according to any one of claims 1 to 11.

13. A computer-readable storage medium, comprising instructions, wherein when the instructions are run on a wearable device, the wearable device is enabled to perform the method according to any one of claims 1 to 11.
